# EUROPEAN PATENT APPLICATION

(11) **EP 1 666 055 A1**
(43) Date of publication of application: **07.06.2006**
(21) Application number: 03796243.8
(22) Date of filing: 14.07.2003
(51) Int. Cl.: A61K 38/43, A61K 31/7088, A61K 39/395, A61M 1/36, G01N 33/68, A61P 35/00, A61P 31/00

(54) **METHOD FOR TREATING ONCOLOGICAL, VIRULENT AND SOMATIC DISEASES, METHOD FOR CONTROLLING TREATMENT EFFICIENCY, PHARMACEUTICAL AGENTS AND COMPOSITION FOR CARRYING OUT SAID TREATMENT**

(71) Applicant: Tets, Viktor Veniaminovich, St. Petersburg, 196066 (RU); GENKIN, Dmitry Dmitrievich, St. Petersburg, 197000 (RU)
(72) Inventor: TETS, Viktor Veniaminovich, St.Petersburg, 196066 (RU); GENKIN, Dmitry Dmitrievich, St.Petersburg, 197000 (RU); TETS, Georgy Viktorovich, St. Petersburg, 191025 (RU)
(74) Representative: Schneider, Henry
(86) International application number: PCT/RU2003/000304
(87) International publication number: WO 2005/007187

(57) **Abstract**

The invention relates to medicine and veterinary science and discloses a novel method for treating oncological, virulent and somatic diseases whose main target for therapeutic action is embodied in the form of DNA which freely circulates in blood plasma (and other liquid media) and originates from tumoral and mutant cells or cells infected by bacteria, fungi or protozoan and from different microorganisms which reside in the organism thereof. Said invention also relates to novel pharmaceutical compositions and to the use thereof for treating oncological diseases and infectious states provoked by bacteria, fungi and protozoa and non-infectious somatic diseases and states produced by accumulation of somatic mutations in cells of organism. Medicinal and immunological compositions, sorption and physico-chemical engineering and the method for using them in order to treat malignant tumors and other diseases are also disclosed.

List of sequences
<110> Tets Viktor Veniaminovich, Genkin Dmitry Dmitrievich
<120> Way of oncological' and infectious diseases' treatment, way of treatment's
efficiency control, pharmaceutical agents and composition for providing the treatment
<210> 1
<211> 485
<212> DNA
<213> Artificial Sequence
<400> 1
acgacggcca gtgagcgcgc gtaatacgac tcactatagg gcgaattggg taccgggccc 60
cccctcgagg tcgacggtat cgataagctt gatatcgaat tctgaccacc ccaaggtggc 120
catccttgtc cctgtgattc cagatctcca gaactggagg tctagcttca gggaaaaccc 180
agattttctt ggcttagccc acctgacagc taatcactgg aaatggggtg ggctggtaga 240
gtcctttggt caggttttgt gtcaagagag ggaggaggaa agatgggagg gaggtagcaa 300
aactggtctc aatggaacta tgtaagttaa tatagaatgg caaagggatg tttcttccaa 360
ggaaagaatt cctgcagccc gggggatcca ctagttctag agcggccgcc accgcggtgg 420
agctccagct tttgttccct ttagtgaggg ttaattgcgc gcttggcgta atcatggtca 480
tagct
<210> 2
<211> 244
<212> DNA
<213> Artificial Sequence
<400> 2
gaattctcaa attattactg aggaaaatgt gacagtgctt caaagcagta gtaatttttt 60
ctcattatgc tgcatttatt attaaaacca acagtggaca gtgaatgact aactgatcct 120
tttttgggaa tattacttcc aaatgaacgt taacttaaag attggaatat gaacacacta 180
ttgcttttac actagagagg ttactcctgg ccactctttc agcagcagtt agcttcagga 240
attc
<210> 3
<211> 230
<212> DNA
<213> Artificial Sequence
<400> 3
gaattcgcag taacttcctt gtgttgtgtg tattcaactc acagagttga acgatcgttt 60
acacagagca gacttgaaac actctttttg tggaatttca agtggagatt tcaattgttt 120
gaggtcaatg gtagaatagg aaatatcttg ctatagaaac tagacagaat gattctcaga 180
aactcctttg tgatgtgtgc cttcaactca cagagtttaa cctttctttt
<210> 4
<211> 218
<212> DNA
<213> Artificial Sequence
<400> 4
gaattctcat gaaattgaaa tggatggact catcatcgaa tggattcgaa tggaatcatc 60
gaataaaatt gattgag(a)at catcatcaaa tggaatcgaa tggtatcatt gaatggaatc 20
gaatggaatc atcatcagat ggaaatgaat ggaatcgtca tagaatccaa tcgaatggat 180
tcattgaatg gaatcagatg gaatcatcga gtgactga
<210> 5
<211> 182
<212> DNA

<213> Artificial Sequence
<400> 5
gaattctcta cagggacaga actaatggaa tatatgtatt atacagggga gtttattaaa 60
cattaactca catgatcaca aggtcccgca ataggctgtc tgcaggcagg ggcgaaggag 120
gccagtgaag ttccaaaact caagaaccta gagtcaatgt tcaagggc(?)a ggaagcatcc 180
ag
<210> 6
<211> 152
<212> DNA
<213> Artificial Sequence
<400> 6
gaattcacag aaatcattgc cacaggcaag atctgatgaa ccttgatgaa tgctaaaatt 60
agttggtgaa agtttaagca gaaacagaat gtttgcatag aatgaagcaa aagaaggaaa 120
aaaaattatg agcccttgat ttaggggtct tt
<210> 7
<211> 131
<212> DNA
<213> Artificial Sequence
<400> 7
gaattcttct gtctagagta acatgaagaa atcccgtttc caacgaaggc cctcaaggcg 60
gtcaattatc cacttcgaga ttctacagaa agagtgtttc aaaactgctc tatcaagaga 120
aatgttccac c
<210> 8
<211> 239
<212> DNA
<213> Artificial Sequence
<400> 8
gaattcccag taacttcctt gtgttgtgta cattcaactc acagagttga acgttccctt 60
agacagagca gatttgaaac actctttttg tgcaattggc aagtggagat ttcaagcgct 120
ttaaggtcaa tggcagaaaa ggaaatatct tcgtttcaaa actagacaga atcattccca 180
caaactgcgt tgtgatgtgt tcgttcaact cacagagttt aacctttctt ttcatagag
<210> 9
<211> 207
<212> DNA
<213> Artificial Sequence
<400> 9
gaattctcta gacttccttg ggtttagcgc tgagtgaaga ggcacggaga gggtttggag 60
ctttagggta aagcactgat ggaagaaagg aattcctgca gcccggggga tccactagtt 120
ctagagcggc cgccaccgcg gtggagctcc agcttttgtt ccctttagtg agggttaaaa 180
gcgcgcttgc gtaatcatgg tcatagc
<210> 10
<211> 223
<212> DNA
<213> Artificial Sequence
<400> 10
gaattcatcg ctaggactgt gttcttgttt attgggatgg gaagggagag aaaagatgag 60
aggggcaaaa gagaaaattt tggaaaatga gaaacttact ttattgcact gtctgtgcaa 120
ttgttggtct taaggaacaa atacactaaa ttcaaagatg ataaaaaaaa aaaacagctt 180
cacagagctg tagtaaacac cagatgttga aagagaagcg tat

<210> 11
<211> 198
<212> DNA
<213> Artificial Sequence
<400> 11
gaattccatt tgatgacaat tccattcaat accaattgat gatgtttatt tttgattcca 60
tttgatgatg attacattcg attccatttc atcatgattc cattcgattc cactcgatga 120
ttccattcga ttccattcaa tgattattcc acttgagtcg attcgatgac tccattcgat 180
tgtattcgat ggtgattg
<210> 12
<211> 217
<212> DNA
<213> Artificial Sequence
<400> 12
gaattctgcc aagcagtgac ttgattcatg aacactcact ggatgctgac tctgttgctc 60
ttctgagtgc tggggtagag gagaggatga ggtggacgca cagttcttgc ttttatgagc 120
ttatgttcta ggaaattcaa acaagtattt tttcaggcag gtagtatgaa atagcaggaa 180
gaggaagcag gctaaaggga cacagagtga ttggggg
<210> 13
<211> 223
<212> DNA
<213> Artificial Sequence
<400> 13
gaattcaggg ctgcagaaat ttgtgtaagt aaagaggagc agaatgttaa tagccaagac 60
aatgcaaaaa atgcattcaa ggtgttttga aaccttcatg gtagcccctc ccattacaag 120
cctggaggnc tgggagggaa aaataatccc tgaaccagga caagggccct atccctattt 180
ctctgtacag tctcaggaca cagcactttg catcccagca gct
<210> 10
<211> 258
<212> DNA
<213> Artificial Sequence
<400> 14
gaattcgctt acagtcagtt acaaatgctt tttagatctt caatgcttct gtgaagcctc 60
atatttgctg ttcagacaga cactataatg gagatggaat aaatggacag caactacaca 120
ggacggtgtg ggcagatggt gttggagcga ggggtgcagg tggagcccac aggagaggaa 180
ggctgattga tcttctatgg ggagagcttc atagcacggg ggtggggcac acctgactgg 240
caagctgttt ggtgtgag
<210> 15
<211> 239
<212> DNA
<213> Artificial Sequence
<400> 15
gaattctttt gaactagctg tgttttgaca gaggtttttt tttttttttt tctttttttg 60
gttttttgct tctctgacaa aggcctttgg aagaatgagc ttcttccccc acatctttat 120
ttatttattt atttttaagc tatgctcagg aaaatgaaca tttctccttt gcagttgata 180
acagcattta caaggtatac agcatatagg gttgttccaa attccttccc agataacca
<210> 16
<211> 226
<212> DNA

[…]

acacagagcg gacttgaaac actctttttg tggaatttgc aagtggagat ttcagccgcg 120
ttgaggtcaa tggtagaaaa ggaaatatct tcgtataaaa actagacaga atgattctca 180
gaaactcctt tgtgatgtgt gtgttcaa
<210> 22
<211> 262
<212> DNA
<213> Artificial Sequence
<400> 22
gaattcaatg gaatggaatg gacaggaatg gaatggaatg gaaaggaatg gagtggaatg 60
gactagaatg gaatggaatg gaatgaaatc aacccgattg gaatggaatg gaatgcaatg 120
gaatggaatg gaatcaactg gaaaggaatc aaatagaacg gaatggaata gaatggaatg 180
gattggaatg gaatggaatg gattcaaccc gagtggaatg gaatggaata gaatggaata 240
aacaacgagt ggaatggaat gg
<210> 23
<211> 218
<212> DNA
<213> Artificial Sequence
<400> 23
gaattcgttg aggagcttct ggaaagtgca cattctgact cagcaggtat tggagtctgc 60
atttctcatg agcactcagg tgatgaaaga gctggtcctt ggacacagct ctgaatagca 120
agggaatagc tttcctttag agaaatctgg aaaaagaacc actggagagc aatttaaaaa 180
ataacagaat ccagggaaag ctttaatttc cttttatt
<210> 24
<211> 213
<212> DNA
<213> Artificial Sequence
<400> 24
gaattcaaag gaatcatcat caaatagaac cgaatggaat cctcattgaa tggaaatgaa 60
aggggtcatc atctaatgga atcgcatgga atcatcatca aatggaatcg aatggaatca 120
tcatcaaatg gaatcgaatg gaatcatcat caaatggaat ctgatggaat cattgaacag 180
aattgaatgg aatcgtcatc gaatgaattg aat
<210> 25
<211> 229
<212> DNA
<213> Artificial Sequence
<400> 25
gaattctgtg cgtattttag aagtagaatt ataagatttg tggatatgtt agttttggag 60
tgtgaggtca aaggcgtttt gagcaacttg taagaaacca tttttaaggc ggaagtcggg 120
aattttgttt tttatatgtt gaatttgaaa tccttattaa acatccaagt ggagaggctg 180
gatagacaat taaatttaga ccctgaggtt cgggaaggaa gtccaatgg
<210> 26
<211> 216
<212> DNA
<213> Artificial Sequence
<400> 26
gaattcttca agaaacatca aggagggatg tatagatagt tttttaaaaa accgaaatgt 60
aaaagaaata caagaagaat ggaaacatct acataacgag agtggaaaga aatgaaaata 120
gaggtagata gattagatag atagatagat agatagattg attgatggat tgatagattg 180
atagatatag aaataaaaga aagaaaatag aagatg

<210> 27
<211> 244
<212> DNA
<213> Artificial Sequence
<400> 27
gaattccaat gcaatgttaa acagaaagca gccctttttt tcaaaattta taggcaaggt 60
gtttaacata tggctaaata atgttaattt atagtaaata tccttcataa ggatgaagat 120
gtacccttct attttagttt gctgagtgtc ttttagtcat aattgagtgt tgacatctgt 180
caaatatttt ttctgcatct attaagacat ccatgtgata tttctctttt attctcttac 240
tatg
<210> 28
<211> 237
<212> DNA
<213> Artificial Sequence
<400> 28
gaattcaatc accatcgaat acaatcgaat ggagtcatcg aatcgactca agtggaataa 60
tcattgaatg gaatcgaatg gaatcatcga gtggaatcga atggaatcat gatgaaatgg 120
aatcgaatgt aatcatcatc aaatggaatc aaaaataaac atcatcaatt ggtattgaat 180
ggaattgtca tcaaatggaa ttcctgcagc ccgggggatc cactagttct agagcgg
<210> 29
<211> 184
<212> DNA
<213> Artificial Sequence
<400> 29
gaattctttc cagaaggttt ttaatttact ttgctcggct ccatcagggg aatcactatg 60
gcagctatag ccttaagaaa tttatttctt aaataagact tgagagtcag aattgcttct 120
ttatccatgg tctcgaggat gggatgttgt gatagcaggc gtgaaaacaa cattcatctc 180
ctgg
<210> 30
<211> 191
<212> DNA
<213> Artificial Sequence
<400> 30
gaattcagaa tctggatggc aaggaagcgc atcaagatgc aggagaaagt tgaaacctaa 60
tccaaggaat acagtaaaac aatccagaag cttgaaagac aaaatagcca ttttaagaac 120
caaactgagc ttctggaagg gaaaaattta cttcaagaat ttcataatac aatcaaaagt 180
attttttttt t
<210> 31
<211> 143
<212> DNA
<213> Artificial Sequence
<400> 31
Gaattccgct tggggaggga actgtcttcg tccaggaaaa tgtttttnat aagccaccca 60
tggtaaaagg agaagtcatg acggttaggg tgttggcagg aatcaaatta agaaaaggaa 120
tggctatcca tccggttgta tgt
<210> 32
<211> 169
<212> DNA
<213> Artificial Sequence

<400> 32
gaattctaga ctgctgcacc tccatatcct cagcaactgg catgatgatg agcagggagt 60
tagtagaact aatacactaa tatgtaaatg aatgaatgaa tgtttcctga gtgtggcttt 120
aagtttctca gaagaagaca gttcatacac tggtgcataa aattctggg
<210> 33
<211> 124
<212> DNA
<213> Artificial Sequence
<400> 33
gaattctagg acaaggtgat tgtcctagat tttctcttaa acgcctcctg ttagatagga 60
aatggccatt aatagagaag cttgcttgag ggagtaaccc tgaaagccca ggcctggaca 120
cccg
<210> 34
<211> 214
<212> DNA
<213> Artificial Sequence
<400> 34
gaattctaag tttatatagg ttacaacatc acagtaagaa tgtcacagag gggtatatgc 60
ttttcatcaa acaacaaatt gaaaattttt taactcttaa ggactgattt tgcttaacta 120
caagttatgc actgatggta gtagcttcat aaatttagaa aagttccaaa ataatgctta 180
gaaagagtag ctatttaact tctcattgaa caaa
<210> 35
<211> 164
<212> DNA
<213> Artificial Sequence
<400> 35
gaattcctgt gaatgtcgtt tcaaatatta ctcagcctac gcactgacca gaacttattt 60
tttacagaat cattttgaca ggaaaagtgt ttatgatagt tttgttgttg ttgttgttgt 120
tttgtttttt catcacccag gctgcttcac atttagagct gagt
<210> 36
<211> 119
<212> DNA
<213> Artificial Sequence
<400> 36
gaattctgag aactagccct ttaagactgg tggagattta ttcaggaggg aagccctgcc 60
ccagggaaaa gttgccaaga gacttgtntt taggagatca ccagcccaaa tttccatga
<210> 37
<211> 208
<212> DNA
<213> Artificial Sequence
<400> 37
gaattccctt catatttttg gtcaaagccc agtttttctg agtcggtggg ctaaatggga 60
ttactctttc taatgaggca tccttgtgtg cttagaatca ctcttgactt tatcctgtcc 120
ccctcgggtt cctaacttac caggatggag agcatttcct cattccatgt tgttgggagg 180
ttggcccact gggtgacatc agcccagg
<210> 38
<211> 169
<212> DNA
<213> Artificial Sequence

<400> 38
gaattcctta acccttaatt agctttggtt tttgctcaat atcctgaagc tgggcacagt 60
ctcaatgtaa ctattctcct aggggctgaa ctgggtgcta gtcatcaaag tttggaatgt 120
cattttagaa gcaacctcta gaagtaatcc tggtaagccc tagaagtaa
<210> 39
<211> 172
<212> DNA
<213> Artificial Sequence
<400> 39
gaattcccat ctttttttgt gtgtgtgttt gagactgtat tttgcattgt cgtccacact 60
ggagtacagt ggcgtgatct ccgctcgctg caagctccgc ctcatggatt taagcgattc 120
tcctgcctca gcctcccaag tggctgggac tacaggtgcc cgaccaacca cg
<210> 40
<211> 137
<212> DNA
<213> Artificial Sequence
<400> 40
gaattctgtt acttggtgat gggaaaccgt gaaggtttta agcaagactg tgatgtgctt 60
aggtttatta gaaggttcta tgctgctcag cctccctgtc tagttctttg ctttattgac 120
tgtntcctca ctaaatg
<210> 41
<211> 152
<212> DNA
<213> Artificial Sequence
<400> 41
gaattctttt ttccccagct ttatggagat ttaattgaca aataaaatgg catatattta 60
ggtgtatata tttgatatat gtatacattg tgaaacgatt actataatga agttaattaa 120
catattcctc atcttgcata gtcaccattt tt
<210> 42
<211> 183
<212> DNA
<213> Artificial Sequence
<400> 42
gaattctcca tgaaaacaga catatttgat atttaggtgc tttaatggac cctgaaaaga 60
aattagattg attcatttga agaataaatg tcggtccccc gccctctaca tggtaaaact 120
cttccaaatg cttctactta atggaaatgg aaattacctc tcaaaacatt acaaaaacta 180
atg
<210> 43
<211> 162
<212> DNA
<213> Artificial Sequence
<400> 43
gaattccgac cactgctgac cgccaggcca cacaccggtt ttnttcagga ggtctcaact 60
agatgctaag ctccgaagtg gaactccctc aggcactttc tgttctaatt caggaattcc 120
tcgagcccgg gggatccact agttctagag cggccgccac cg
<210> 44
<211> 189
<212> DNA
<213> Artificial Sequence

<400> 44
gaattctgtg aaataattct cagcccagac ccaaggg(a)tc cacagctcag aaataggtta 60
tccagaagtg ttcctaacac tagatgacag tatcccagtg ctccaaacca gcttattact 120
tggccagaat tcctgcagcc cgggggatcc actagttcta gagcggccgc caccgcggtg 180
gagctccag
<210> 45
<211> 190
<212> DNA
<213> Artificial Sequence
<400> 45
gaattctctg tctgtcgatt tcagtgattt tagtgctggt cctccacttg agtactagcc 60
ataggtcttg gcttggcact cccatcccat agccctgtgc accatagctc tggggtgaac 120
tcaggcaaaa cgattttcgt ccccagcttg ggagcagcag ggttggggac cttggcaatg 180
gcaatggcag
<210> 46
<211> 266
<212> DNA
<213> Artificial Sequence
<400> 46
gtaatacgac tcactatagg gcgaattggg taccgggccc cccctcgagg tcgacggtat 60
cgataagctt gatatcggct tatcctgagc taggctgagc ctttgctctc ctgacctagt 120
tagttctcat tcaaccctgt gacaagggat gtggggctca gagaacggga gggtcttccc 180
tcaggtcaca tggccagggc atggagaggc aggacttgaa tccaggtcaa tgtgacccca 240
gagcctagtg tggaaacccg tccttt

<210> 47
<211> 164
<212> DNA
<213> Artificial Sequence
<400> 47
gaattctacc ctgggtagga tagtagctcc cctcaacttt acagcaaata cagctaacct 60
tgctttacct gcgatcccgt ntttattttg ttgaattaga gaaactgagg gaagcagttc 120
tctacactca ctttaccctt agagccctct acaatcaacc ctgt
<210> 48
<211> 112
<212> DNA
<213> Artificial Sequence
<400> 48
gaattcaaag actgtnagat gtaagcagtg actganacan aggcaatgag atgagaggtg 60
gaaaggagac caaatgtaaa agacagcaga aacttgagtg gacggtggca ca

<210> 49
<211> 114
<212> DNA
<213> Artificial Sequence
<400> 49
gaattctgtt ggctttacct ttaacgtgtc caaaagtgac caattatcat tnctgcnttt 60
ngctgctact tggntcaagc cattagtatc ccttgctcca ataaactctt tcct
<210> 50

<211> 206
<212> DNA
<213> Artificial Sequence
<400> 50
gaattcccag taacttcctt gtgttgtgtg tgttcaactc acagagttga actttcattt 60
acacagagca gatttgaaac actctttttg tggaatttgc aaatggagaa ttcctgcagc 120
ccgggggatc cactagttct agagcggccg ccaccgcggt ggagctccag cttttgttcc 180
ctttagcgag ggttaattgc gcgctt
<210> 51
<211> 169
<212> DNA
<213> Artificial Sequence
<400> 51
ccccccctcg aggtcgacgg tatcgataag ctggatatcg gcaacttctc gctctgtcct 60
cacataggga aagaggaagc tgttgccttc ctcttacaag agcactaatc tcacatgggt 120
gtttaccctc atgactttat ctaaacctaa ttatctttca aagaatcta
<210> 52
<211> 141
<212> DNA
<213> Artificial Sequence
<400> 52
gaattcttgt tttcagtgaa aatttagata atttatctca ggaattcctg cagcccgggg 60
tttccactag ttctagagcg gccgccaccg cggtggagct ccagc-t-tg ttccctttag 120
ttaggg-taa ttcgcgcttg c
<210> 53
<211> 203
<212> DNA
<213> Artificial Sequence
<400> 53
gaattctata tatttcccct cttttcctga ctcttcagtg acaatcctaa gaccgtgcta 60
ataacagaag acagtaatcc ctttttttag ccaaataatt tggaagccat gattttcttt 120
gcatatcatg aaagtgacca tggtgttgga tattgtgggt agaagctttc aagtaaaaaa 180
gaactgtcat tcaactgaat tgg
<210> 54
<211> 162
<212> DNA
<213> Artificial Sequence
<400> 54
gaattctagg ccaggcgcga tggttcacac ctgtaatccc agcattttcc cgggaagcca 60
aggcaggcag atcacttgag gccaagagtt caagaccaac ctggccaaag gggtgaaatc 120
catctctact aaaaatacaa aaattagtcg ggcgcggcgg cg
<210> 55
<211> 193
<212> DNA
<213> Artificial Sequence
<400> 55
gaattctatt tctaggaacg ttctcaaaca agcttaagag caaagtataa aaacgatgtt 60
cagcatataa taatatgaaa aaattttgtc ctagacattt tatatgaaaa tgtatacttt 120
agagcatgct tcaggaaaaa aagaaagaaa aattaatcct gggaaatggg tgacattaga 180

tacaggcgag tgg
<210> 56
<211> 169
<212> DNA
<213> Artificial Sequence
<400> 56
gaattctgct tttatgagaa gtcagctgaa tgctatggaa aggagtatag agagtggctt 60
aaaagtttca ggcaagttca caccaaaact tgcattctaa cctccctgaa cctgtggtct 120
agaagggacc tatcagcaag atgataacca aaaatgtcta gaatctgag
<210> 57
<211> 141
<212> DNA
<213> Artificial Sequence
<400> 57
gaattctaga gaacaatccc tactgacttc acacacaact taagaaatgc aagtaaaggg 60
ccgggcgcgg tggcccagca cctgtaatcc cagtactttg ggagcctaga ggcaggtggt 120
cattggaagt caggagttca a
<210> 58
<211> 183
<212> DNA
<213> Artificial Sequence
<400> 58
gaattctctg atgtttagtt aggtatgacc tacagttaaa ggctttgctg cattccttac 60
gtttgtaggg tttctctccg gtatgactac ttcgatgtcg agtaacggac gttgaattac 120
gataaaaggc tttgccacat tctttgcatt tatagggttt ttctccagta tgaattccag 180
cag
<210> 59
<211> 185
<212> DNA
<213> Artificial Sequence
<400> 59
gaattctatc aatgtcaatt aaatccagtt gatggatggc cataatttta aatctattta 60
cattttgggg tattttttaa aataaaatct gtgattatct atcttttaat gaatgcctta 120
gatcattcac attaaagtga ttgttgttgt agttgtgttc atgtatacca tacttataac 180
tgttt
<210> 60
<211> 163
<212> DNA
<213> Artificial Sequence
<400> 60
gaattctact aaaactttag aaaagaaatt aacaccaatt ctcaaactat ttcagaaaat 60
tgaaaaggag aagcctctcc caactaattc tatgaatcca gcattacccc ttaccaaaac 120
cagacaaaga tgaaacaaaa taataagaag aaggaactct ggg
<210> 61
<211> 103
<212> DNA
<213> Artificial Sequence
<400> 61
attttccctg ctgggtgtgt ccagagatcc tttctggcta gtctgctagc actgcatgtg 60

tcnaccagca tctcaacctc acactagctg caacacttgg cca
<210> 62
<211> 144
<212> DNA
<213> Artificial Sequence
<400> 62
cctctccaaa aagaaaatct ctgccattct atgtacactg gctgcatgaa gatgtatgtn 60
tatgaattag cctgcatgtc tgggtcccac cctgcacatg ctaacattcc tttccctccc 120
catacgagtc caaaaaaact atgc
<210> 63
<211> 173
<212> DNA
<213> Artificial Sequence
<400> 63
tcagtcttca ggtgatattg aaatggaggc tgtaaggttt taataataca ggtttcaaaa 60
ccaggcagca acacatacta gccatgtaaa acttgagcta ccccaacccg cctggttgtt 120
gcttagtcct tctttgaaaa ttaaaattct gttctctgga aatagtattt agg
<210> 64
<211> 150
<212> DNA
<213> Artificial Sequence
<400> 64
ttacaacctt tatgagattg gtgccattat caccattttc agacatgaaa aatacagcac 60
acacagttta agtaatatgc tgaattcctg cagcccgggg gatccactag ttctagagcg 120
gccgccaccg cggtggagct ccagcttttg
<210> 65
<211> 159
<212> DNA
<213> Artificial Sequence
<400> 65
ccagtaactt ccttgtgttg tgtacattca actcacagag ttgaacgttc ccttagacag 60
agcagatttg aaacactctt tttgtgcaat tggcaagtgg agatttcaag cgctttaagg 120
tcaatggcag aaaaggaaat atcttcgttt caaaactag
<210> 66
<211> 73
<212> DNA
<213> Artificial Sequence
<400> 66
tcccaatcct tcctgtgact caagcntctg ctcattaggt atcctaggac aatattatgc 60
tgtntctatc aga
<210> 67
<211> 87
<212> DNA
<213> Artificial Sequence
<400> 67
agccagagcc aagctctctc actctgcaga gaagcctcag tctttagaag acagttcagc 60
tttatccaga attcctgcag ccggggg
<210> 68
<211> 110

<212> DNA
<213> Artificial Sequence
<400> 68
tatgatcaac aaatatatct tacaacatga gggtgcaata agatgagaaa ggttcgagag 60
tgtttatctt tagcaaatac atactatcgc gctcaaggta agtnttcaag
<210> 69
<211> 111
<212> DNA
<213> Artificial Sequence
<400> 69
Tattgtgccc agagataatt gtcctgcagt cagagcattc tatgtntttn tctgtcgttg 60
attaatcaag agggtttcag gcttccctgt aggaaaatgt ctaaagcata a
<210> 70
<211> 138
<212> DNA
<213> Artificial Sequence
<400> 70
attcatttat accctcattt attcatccaa cagccattca ataagcgtct gtgttcagcc 60
atgctctgac actgattgan ttcctgcagc cgggggatcc actagttcta gagcggccgc 120
accgaggtgg acgtcagc
<210> 71
<211> 144
<212> DNA
<213> Artificial Sequence
<400> 71
caggttgatg aagaaacgga tattagtgca atgaagaaca gctccgtctc tgtcagctgg 60
tcatttttta tatgtcagag actgtcgaat ttctattgcg tttcaactaa ttacctcagt 120
ttgttaaaac tgaatatgaa ttcc
<210> 72
<211> 113
<212> DNA
<213> Artificial Sequence
<400> 72
ntctatctag ttttatatga aganatcacg tatcacacga tggacccaaa gaggtccaaa 60
tatccacttg cagttctaca aaaagagtgt ttcacaacag cactatcaag agg
<210> 73
<211> 97
<212> DNA
<213> Artificial Sequence
<400> 73
tacattcttt ttcttaacta tccaccacct cccctcaaaa ttttaacagc atccagcctc 60
acaaaactca gatcttccct gtgtacagtt ccacttt
<210> 62
<211> 143
<212> DNA
<213> Artificial Sequence
<400> 74
gacaattcca ttcaatacca attgatgatg tttatttttg attccatttg atgatgatta 60
cattcgattc catttcatca tgattccatt cgattccact cgatgattcc attcgattcc 120

attcaatgat tattccactt gag
<210> 75
<211> 98
<212> DNA
<213> Artificial Sequence
<400> 75
aaatgataat atagtcaatt caggaaagan aatcatccta anatttcgta ttatgattag 60
aagtgtaatt tcgctganat agaaaatttc tcattatt
<210> 76
<211> 88
<212> DNA
<213> Artificial Sequence
<400> 76
agctgacatt gtaatttaat aaagctaagg ataaaacttc tgggtttttt gtttattgag 60
cccgctgact agaagagata agagatgg
<210> 77
<211> 101
<212> DNA
<213> Artificial Sequence
<400> 77
ctctggttgt tgtcaggttt ttnattatta gattccagaa ttcctgcagc ccggnggatc 60
cactagttct agagcggccg ccaccgcggt ggagctccag c
<210> 78
<211> 109
<212> DNA
<213> Artificial Sequence
<400> 78
aaggttacag tgagctatga tccaccactg cactccagca tgggcaacaa agcgagaccc 60
agtatttaga tttatttgtt aatagccagg catattggta catgcgtgt
<210> 79
<211> 121
<212> DNA
<213> Artificial Sequence
<400> 79
ctatatcaca tactttattg tcttgtacag tttgctttgt ttcatgtgtg gataccctga 60
nttcctgcag cccgggggat ccactagttc tagagcggcc gccaccgcgg tggagctcca 120
g
<210> 80
<211> 144
<212> DNA
<213> Artificial Sequence
<400> 80
ctatgagtgg cctccaagga gcattagatt agaaggtggc tggagggtgg atattttcat
acacagagac aaagctcccc atcccacaac agatccagag tctgtnttgg accacaggga
aggaaggccc ttctccagga ttct
<210> 81
<211> 160
<212> DNA
<213> Artificial Sequence

<400> 81
ttaggagagg tcagagtggg ctggagcagc caggtgagcc tttgttgtgt aggcaggagg 60
aagaagcagt ggattttgag ttgaggacgg aatttgagag ggggagggaa aaggaaggga 120
atccgcagag gcagagctga ctgcactcgt gagggagggg
<210> 82
<211> 164
<212> DNA
<213> Artificial Sequence
<400> 82
atacaaattg cagactgcag cgttctgaga aacatctttg tgatgtttgt attcaggaca 60
gagagttgaa cattccctat catagagcag gttggaatca ctccttttgt agtatctgga 120
agtggacatt tggagcgctt tcaggcctat gttggaaaag gaaa
<210> 83
<211> 164
<212> DNA
<213> Artificial Sequence
<400> 83
ttgtggttct agattttatg gtctcttttt tatttttcat tttttgagac caagtttcac 60
tcttgttgcc cggctggagt gcagtgacgc gatcttggct caccgcaacc tctgcctcca 120
ggattcaagc gattcgcctg cctcagcctt actgagtagc tccc

<210> 84
<211> 141
<212> DNA
<213> Artificial Sequence
<400> 84
tagttccagc tataccactt tctagccttc ttgattttgc tgaactgaga gtcagaagag 60
atatgtntct aggttatttc caatcattat gccatctcgg aagtggcagg ggtgctatac 120
tagactgaga caaatacccc a
<210> 85
<211> 72
<212> DNA
<213> Artificial Sequence
<400> 85
cttctaaaat tctatggtag tatganaggc tacacaaaag tntttggacc tgatacaaat 60
attataaatg at
<210> 86
<211> 135
<212> DNA
<213> Artificial Sequence
<400> 86
tcataaaata accattaata tttcactttc gttttttatc ctaacctttt tctaacacat 60
aaacatattc attgggaggt cgaggcgggc ggatcacgag gtaggagatc gacgaccatc 120
cggtaaaagg tgaaa
<210> 87
<211> 107
<212> DNA
<213> Artificial Sequence

<400> 87
   cagccccaag aatgtctgga gcccgagtat catctggcag ccaccctcgg agaagggggg 60
   gatccactag ttctagagcg gccgcaccgc ggtggagctc agctttt
<210> 88
<211> 109
<212> DNA
<213> Artificial Sequence
<400> 88
ccatgtggaa gcacagctat aaggctcttt ctatgaacca gaaagcaggc tttctctaaa 60
caccgaatct gccaatgcct tgatcttgga tttcccagat tccgaacta
<210> 89
<211> 112
<212> DNA
<213> Artificial Sequence
<400> 89
cagggactta atcaacgcaa gcttatgacc cgcacttact gggaattcct cgttcatggg 60
gaataattgc aatccccgat ccccatcacg aatggggttc aacgggttac cc
<210> 90
<211> 125
<212> DNA
<213> Artificial Sequence
<400> 90
acctgtaatc ccaactactc tggaggctga ggcaggagaa tggcatgaac ccgggaggtg 60
gaggatgcag tgagccaaga ttgtgccact gaactctagc ccaggcaaag gtgagagact 120
tgatc
<210> 91
<211> 130
<212> DNA
<213> Artificial Sequence
<400> 91
cacttaagat tgtatctttn actctatgag ttatttctca ataaaaagta aaattnannn 60
tactaataat taganatnat cttctctaga atgagcattn aatgagtcag ctagagaggc 120
gacttaactg
<210> 92
<211> 104
<212> DNA
<213> Artificial Sequence
<400> 92
cagcccttac attgtgtctg tgacccagtg ttaaatgaga cccaggtcaa gagacaactc 60
tttggctggt ctaggatatt ntataanata gatctatcac tctg
<210> 93
<211> 122
<212> DNA
<213> Artificial Sequence
<400> 93
cgtcagctca gcagcctgac aatttgaact cagtagtatc acattgccac atggctatgt 60
tcaggggtta atacttctta gcaaagaaat agagaccaat ctctgtgatc actttaaact 120
tt

<210> 94
<211> 76
<212> DNA
<213> Artificial Sequence
<400> 94
cacatggatg gggaggcctt ccaatcatgg cagaaggcaa aggagaaagn nagcacatct 60
tacaggcagc aggcaa
<210> 95
<211> 109
<212> DNA
<213> Artificial Sequence
<400> 95
cagccccagc atggcaggaa tatntntngc attgggttct ttggaggagg aaagtacgtn 60
ctcagagnag gcaatttntc gccgctggtt taaggctttn natgaccga
<210> 96
<211> 112
<212> DNA
<213> Artificial Sequence
<400> 96
cagccccgaa ttatgtatta anagttatcc tcaccaagaa agacaaggtt tctgtagttc 60
tctaacatca tatccctata tanntntnac tgtgcagtat ccagacaatg acactccttc 120
agagagaatt ctatggccac atctctaa
<210> 97
<211> 122
<212> DNA
<213> Artificial Sequence
<400> 97
taaaactttg ttataagaga tggaagggtt taaatatata nntctaannn nttntagttt 60
aaagaattcc aaacttaaac atcttcagta gacttgacat tgtatttcgn atatcctatg 120
tc
<210> 98
<211> 88
<212> DNA
<213> Artificial Sequence
<400> 98
ctttaaattt ataaactcca aggcagtaca agtctggnnn nnnnnnagct acccaatatc 60
tgataaatat gaatacctaa taatagac
<210> 99
<211> 105
<212> DNA
<213> Artificial Sequence
<400> 99
tcctaaaact ctccctcacc agcatcccaa tttaaagcct tggtccttgc tcctccctct 60
agggggatcc actagttcta gagcggccgc caccgcggtg gagct
<210> 100
<211> 86
<212> DNA

<213> Artificial Sequence
<400> 100
cgccactatg ctcagctact tnnnntntgt tttgtagaga tgggtgtttc accatgttgc 60
ccagactgat cttnanctcc tgggtc
<210> 101
<211> 156
<212> DNA
<213> Artificial Sequence
<400> 101
gaccctccac tgatttncca tcttgaccac tgcctaccca attactgtnc cagtcgaaac 60
ctgggcgcca tgtgacgact ctctccctct ctacagctac acaaccgccg tgtgctgtcg 120
ggtcttatcc tttccaccca gtccatggct tggtct
<210> 102
<211> 173
<212> DNA
<213> Artificial Sequence
<400> 102
cagccccata aaattaacca tcacactagg tgatgtcttt nttttttgag agcaagtctt 60
gctcgtcacc aggctggaat actgtggtgg gatctcagct cactgcacct ccacctcctg 120
ggttccagca attgttctgc ctcagcctgg gggatccact agttctagag cgg
<210> 103
<211> 191
<212> DNA
<213> Artificial Sequence
<400> 103
cagccccctt agaaatagct cttcgagaca ctcctggtag acatgatccc aggcttgctg 60
agcagctgtg caaccatgcc tcaggcctga ggaacagctc gcaggccact ctgtctggta 120
ataccccagg ccggccaagc aatagatctg catcccaggg ggatccacta gttctagagc 180
ggccgccacc g
<210> 104
<211> 191
<212> DNA
<213> Artificial Sequence
<400> 104
6agcccccctt ggctcagtct ggaaaggcaa gacaactaga aggtgggggg cttccagggc 60
ataggtagat tcanaaatgt actgattggc acttccttga ccgagttatt aactaaagac 120
ctggaatcaa tagaaaggaa tgtctgggtt aaggtaaggg ctatggggga tccactagtt 180
ctagacggcc g
<210> 105
<211> 103
<212> DNA
<213> Artificial Sequence
<400> 105
ttctnagana tttnacatca nattaaccca ctganaaact tgcnaactct cactttcaac 60
gtctgancgg naattttaat tggnggatcc actagttcta gag
<210> 106
<211> 173
<212> DNA
<213> Artificial Sequence

<400> 106
cagccccctt attaactcac cccttgcatt tgttcaaccc tagntaataa agtcactcag 60
gtgtacttct ganaattgaa gttaaatatt tttcaccaca gagctgaacc attacagagg 120
<210> 107
<211> 111
<212> DNA
<213> Artificial Sequence
<400> 107
tcataanata accattaata tnnnnntnnn nnnnnnatcc taacattttt ctaacacata 60
aacatattca cttgggaggc cgaggcgggc ggatcacgag gtcaggagat c
<210> 108
<211> 70
<212> DNA
<213> Artificial Sequence
<400> 108
caatttacac tctggcaggg ggaganagga naatttntnc tgtnggaagg gggagttgng 60
gnaggaggcc
<210> 109
<211> 104
<212> DNA
<213> Artificial Sequence
<400> 109
caaanactaa natacctctn agtctggnta gacactttca ctggataggt agaggccttt 60
nctacaggnt atnanaaggc caccacagtc atttnttccc ttct
<210> 110
<211> 68
<212> DNA
<213> Artificial Sequence
<400> 110
tcatgtaggc ctnttcacga ttttnnaaat catttnagtn acatccaagt nnnnntngct
gttaatca
<210> 111
<211> 107
<212> DNA
<213> Artificial Sequence
<400> 111
cagccccaat caagggctgt ttctcaatct ctttgtataa aannctagat tctgtattag 60
tctgttctca ggctgctaat aaagacatac ccaaggctgc gtacttt
<210> 112
<211> 173
<212> DNA
<213> Artificial Sequence
<400> 112
tggaaagaaa aactatgtac atctgagacg ctgcagctgg tatcctactt ctttcagagc 60
atcaacaggt taagtgtgga ttcatccaca ccctcagacc cgtgaccgta g
<210> 113
<211> 121
<212> DNA
<213> Artificial Sequence

<400> 113
gaatctctac accaaccctc tcttaacctc tacagttcaa atccaaatct caaactttct 60
gatttgaatt tgcttatccc tatgtaattc taacttaaga cctaagacca aaagggaatc 120
c
<210> 114
<211> 103
<212> DNA
<213> Artificial Sequence
<400> 114
tcttccagct aaatagttgc agagtcagag tagaagccag ctctcctgac aatatatttn
atgatattct agagaatatc cctagaatca ttcctaggta ctc
<210> 115
<211> 86
<212> DNA
<213> Artificial Sequence
<400> 115
tgtcattggt aatttatgtg agaacacaaa gcatccaaca ntanntgatt ctgcatttcg 60
accaacagat agtttctcat cgaaga
<210> 116
<211> 120
<212> DNA
<213> Artificial Sequence
<400> 116
cagccccgtt tgttttacct ttngcttttn atgtgcttct ctaacanttn agggcgaact 60
aaccagcatg aggnttgtnt ctgcttgatt ttnaaccatc ctttcctgtc tgtacacagg 120
<210> 117
<211> 95
<212> DNA
<213> Artificial Sequence
<400> 117
ccctccctga gtctntntaa cagcagcact gcccccaaac ctnanttggt tcccctgata 60
gccaggtacc cggnttctnt ngcagtgcta actgt
<210> 118
<211> 109
<212> DNA
<213> Artificial Sequence
<400> 118
tattaannnn nctaatctna atattntngt ntctcgggga acagaaaagc ctgaggagaa 60
ggagagatag tnggaatntc tagttnttgg agcagtcaga acacacata
<210> 119
<211> 79
<212> DNA
<213> Artificial Sequence
<400> 119
cctgtattac agaaccaagg attaaaaact cagcagatgt gtaatgagtt ttaaataatt 60
acaatatnnn nnntataaa
<210> 120
<211> 83
<212> DNA

Artificial Sequence
<400> 120
tagttgatcc gnnagcccat gcgataccgc gnnggcgctc gnngccgang ggggatccac 60
tagttctaga gcggccgcca ccg
<210> 121
<211> 177
<212> DNA
<213> Artificial Sequence
<400> 121
cgtttgtttt acctttcact tttaatgtgc tttctctaac aattaagggc gaactaacca 60
gcatgaggat tgtgtctgct tgattttaaa ccatccttta atgtctgtac acaggaaatg 120
ttatcaacaa gagatgattc ttgggggatc cactaggttc tagagcggcc gccaccg
<210> 122
<211> 103
<212> DNA
<213> Artificial Sequence
<400> 122
ttatagttta anacanagat ggtaacagcc ctttcccaaa gcagacctcc ttcttgcctg 60
gnaaagggct gttaccatct ttgttttaaa ctataaacta taa
<210> 123
<211> 139
<212> DNA
<213> Artificial Sequence
<400> 123
caagaagggt ggtgctggca tttncttctg gtgagggcct caggaagctt tcaatcatgg 60
cagaaagtga gaggagagta ggcatgtcac anagagagac atgccttcat tctcggggga 120
tccactagtt ctagagcgg

<210> 124
<211> 103
<212> DNA
<213> Artificial Sequence
<400> 124
cattaaagcc tttnttagga aatctnttta aacaacagaa taaaagggat gactttnaga 60
tagaactttn ngtgacatct ccagtttctg gttacatgat att
<210> 125
<211> 103
<212> DNA
<213> Artificial Sequence
<400> 125
cagagagaga gaaacanaca gncagagaga gagagaccac anagagagag agagagagaa 60
gatcagacag agaaaganag agacagagac agacannnag aca
<210> 126
<211> 113
<212> DNA
<213> Artificial Sequence
<400> 126
cagccccaga gagagagaaa cagacaggna gagagagaga gacacagaga gagagagaga 60

gagaagatca gacagagaaa gagagagaca gagacagaca nanagaatag aga
<210> 127
<211> 181
<212> DNA
<213> Artificial Sequence
<400> 127
actcatttta tgaggccaga atcatcctga taccaaaacc tggcagagac acacacacac 60
aaaagaaaat ttcaggccaa tatccctgat aaacattgat gcaaaaatcc tcaataaaat 120
actggcaaac tgaatccagt agcacatcaa aaagctgggg gatccactag ttctagagcg 180
g
<210> 128
<211> 150
<212> DNA
<213> Artificial Sequence
<400> 128
cccgccccat gtagctctca ggtggcccat gacaccacac tgttcttcct tcctctccat 60
gggtcacacc ggccacctag tcagtcctaa cgtcggaacc tggatacctc cattgctggt 120
gctggacccg tcactgtttt ggatattttc
<210> 129
<211> 173
<212> DNA
<213> Artificial Sequence
<400> 129
tcctaagtgt cccaacagtg gagcacatta ttcaggaact taaagatata atcgcagaac 60
agcacctcca agctcgtaaa tgcttatctc ggtaaccctc agtcatggga caatcaaatt 120
caatacatcg gaggaacacc atgctgacgg gggatccact agttctagag cgg

<210> 130
<211> 187
<212> DNA
<213> Artificial Sequence
<400> 130
ctatagaagc tccttctata ttnngcttat nncactcatg gcggtagtft gaattcagat 60
ctctgggtca tttattatcc atggaaagtt aatttgagat gttggaactt ttaaacagtg 120
tttgtttatt gtgctaatca cgatctgtta ctaaatttga ttgggggatc cactagttct 180
agagcgg
<210> 131
<211> 170
<212> DNA
<213> Artificial Sequence
<400> 131
cagatatttg tagatatgcc gcgttatttc tgagggctct gttctgttcc attgatctat 60
atctctgtct ttggtaccag taccatgctg ttttggttac tgtagccttg tagtatagtt 120
tgaagtcagg tagcatgatg cctccggggg atccactagt tctagagcgg
<210> 132
<211> 147
<212> DNA
<213> Artificial Sequence

<400> 132
tctctaaaat tctatggtag ttgaaaggct acacaaaagt ttttggacct gatacaaata 60
ttataaatnn nnnnnnnnnt gtntgatttg atactccatg taaaactctt cctaatggtc 120
tcgggggatc cactagttct agagcgg
<210> 133
<211> 123
<212> DNA
<213> Artificial Sequence
<400> 133
tattaaaaat acaaaaaatt agccgggagt ggtggcacgc gcctgtagtc ccagctactc 60
gggaggctat ggcaggaaaa tcccttgaac ctgggaggcg gaagttgcag cgagaagaga 120
tca
<210> 134
<211> 164
<212> DNA
<213> Artificial Sequence
<400> 134
ctgtctttca agtttcaggc ttgaaagtga aaaataatgc ataatttacg gaagctattg 60
gtgtgaaaat atccaagaga agaatgagga atagtggagt gaaataaaca ggagattagg 120
tagatagaaa ttgactattg ggggatccac tagttctaga gcgg
<210> 135
<211> 193
<212> DNA
<213> Artificial Sequence
<400> 135
cttaatatgg tatgcttaat gtagtgagct aaacaaaata acaatgtgta tagtattgtn 60
taanataccc cacttccaat tgtttaaagt gcaaaacaaa ttatatgttt ganagttaag 120
gtggaataaa tgaagattaa atgatatgaa ctactcagaa aacaggtagg gggatccact 180
agttctagag cgg
<210> 136
<211> 233
<212> DNA
<213> Artificial Sequence
<400> 136
cattgattaa atttattgat gcattgtaaa tttgaatcaa tatctattaa tcccaagctg 60
gagtgcagtg gcgccatctc agctcactgc gacctctgcc tcccgggttc aagcaattct 120
catacctcag cctcccgagt agctggaacc acaggcatga gccaccatgc ccggctagtt 180
acagggtttt cctatgctat ccaggctgga gtgcagtggg ggatccacta gtt
<210> 137
<211> 194
<212> DNA
<213> Artificial Sequence
<400> 137
ctaaaggatc cttcaactct gtgagttgaa tacacacaac acaaggaagt tactgagaat 60
tattctgtct agcataatat gaagaaatcc cgtttccaac tgaagacctc aaagaggctg 120
aatatccact tgcagacttt acagagtgtt tcctaactgc tctatgagag ggggatccac 180
tagttctaga gcgg
<210> 138
<211> 155

<212> DNA
<213> Artificial Sequence
<400> 138
cagcccggaa aatatagggc aaattttttt attttgctgt ttggtgactc caccactttt 60
gcaacagtac ttttggtgcc cattaaccaa attactttga tttctttgtg taaatattat 120
gaagaccaga accttttgag ggggatccac tagtt
<210> 139
<211> 200
<212> DNA
<213> Artificial Sequence
<400> 139
ctagacaaaa gccccatcac ctggatgaat cagtgcagag ttacgtcaca aagtcctttt 60
aggcagatcc tagacaaggg ttacatcact tggatgatca gtgcagagat atgtcacaat 120
gccactgtag ggtgagccta gaaaagagtt tcatgaccta ggtgatcagt gcagaggggg 180
atccactagt tctagagcgg
<210> 140
<211> 169
<212> DNA
<213> Artificial Sequence
<400> 140
ctgtgactgt gcctatagaa gaaaaaaaaa atagcgtgta atctcagcac tctgggaggc 60
caaagcaggg gggatcactt gaggccaaga gttcaagacc agcctggcca acaaagcgaa 120
accttctctc tactaaaaat acaaaaatta gccgggcatg gtggcactc
<210> 141
<211> 211
<212> DNA
<213> Artificial Sequence
<400> 141
agggccacca gctggtgaat cctgccccac cagctcagag ctcttcccat tcatggagta 60
tatcatagga gactggattt ccaaagctgc atggagcttc attcctgaac tggtcaccct 120
gtgtctagtc ttgttttctc aatccatcct gctctccagc agcctcaata cttctaaaat 180
tgtccggggg atccactagt tctagaggcg g
<210> 142
<211> 195
<212> DNA
<213> Artificial Sequence
<400> 142
   cacagacatc ctgtgccacc tcattcactc tcacatgcct ctgaggtgag ggggataaca 60
   gcactagtat catttgatac tgatacaaat cggctctaaa tattgtgggg atgctggtgg 120
   tgttattgct ggactccatt acacaagttt catgagccag tgaaaatcac tgtgggggat 180
   ccactagttc tagag
<210> 143
<211> 199
<212> DNA
<213> Artificial Sequence
<400> 143
   Cagccctaaa gtataataaa aaaaaatttt ttaaagaatc ttcacaaaag aactctgaaa 60
   tgtcagcatg agcagatgat gaagtatcat aggaatccat tttttgctgt atttcttatt 120
   taatagagaa agaaatttca tatgctgtaa tatgtttcca attggaaatt aaaatctgat 180
   aggggggatc cactagttc
<210> 144
<211> 178
<212> DNA
<213> Artificial Sequence
<400> 144
cagcccccct gtaacaatat gggctgttct agctgtaatt cacctctgga gccatcagaa 60
tcctcctggt aaaaatggcc ctaatatcaa acacagaggc cactgctagt taaactttat 120
aaatcgaaca agaaatcata tgatataatc agataagagc ctgggggatc cactagtt
<210> 145
<211> 158
<212> DNA
<213> Artificial Sequence
<400> 145
cagccccctg ggctcaagca atctgcccac ctcggcctcc ccaagtgctg ggattacagg 60
tgtgagtnac tgtncccggc cagccttgtc tatttgtcag aaacagggag ttggggcaac 120
cctggtgcca agatatgggg gggatccact agttctag
<210> 146
<211> 184
<212> DNA
<213> Artificial Sequence
<400> 146
cagcccctgc taaataactt tcgaagttaa gaaagctaat ggtatatcat caggcaccaa 60
taaaactatc ttgagatttg acaatgccaa ctgaaaaatt tcttctgcaa ggcagagcca 120
gttacctttt ataatatcaa tttagattca cacaaagaca ttctcagggg gatccactag 180
ttct
<210> 147
<211> 219
<212> DNA
<213> Artificial Sequence
<400> 147
cagccccacg ggtggtaatc ntggctgctt tntgcacttc cacataaagt gcttctncta 60
cgctgtctcc actcagaaac aattacaaca gtatgtgaag cagtattgaa aacttcnnaa 120
gctgcacaca gattcattga aaagggcaga agcctcatta atactagagt ctgaggcaca 180
acctatgacc gaacactggg ggggatccac tagttctag
<210> 148
<211> 185
<212> DNA
<213> Artificial Sequence
<400> 148
cagcccccag aaaaaaaaga gcaagaggat ggggctgaaa aaattactca aagaaataat 60
ggctaaaaag tactcaggtt tatcaaaaga caagtctgca gaactaagaa gatgacaaaa 120
tccttgtcat agacagaatg tgtgtttccc aaacttcgtg tgttgggggg atccactagt 180
tctag
<210> 149
<211> 129
<212> DNA
<213> Artificial Sequence
<400> 149

cagccctgca gtatttagtt ttctattcct gagttagttc acttaggaaa atggtctcta 60
gctccatcca tgaagcacca aatccctcca gcccagtagc aaggagacag aatttttact 120
ctgtctctg

<210> 150
<211> 74
<212> DNA
<213> Artificial Sequence
<400> 150
cagcccctct tttctgctcc taaggaagat gcattctcag gatacaggan nnngggggga 60
tccactagtt catg
<210> 151
<211> 95
<212> DNA
<213> Artificial Sequence
<400> 151
cagccccatt taacctggag aggaataccc taaggattct tggaggctga aagacttaaa 60
atttgaggaa tgaaagaata gcaagggtga atcgg
<210> 152
<211> 144
<212> DNA
<213> Artificial Sequence
<400> 152
cagccctgca gtatttagtt ttctattcct gagttagttc acttaggaaa atggtctcta 60
gctccatcca tgaagcacca aatccctcca gcccagtagc aaggagacag aatttttact 120
ctgtctctga tgagaagagt gtac
<210> 153
<211> 138
<212> DNA
<213> Artificial Sequence
<400> 153
cagccctgat agttacctta ctgttttgct atgaccatac tctacataga gtatttagat 60
taaatggagg aatgagaata tgagattagt ttctcatatt cttgtgatca tgacaggacc 120
tgagattctg cacagatg
<210> 154
<211> 139
<212> DNA
<213> Artificial Sequence
<400> 154
cagccccgct gtttctaaag tcagtgtgtg tgtgtgtgtg tgtgtgtgtg tgtgtgagag 60
agagagagag agagagagcg tatgcatgtg tgtctgcatg tgtgtgtgcg cgcgtacatt 120
tgggagacgg tgtgtaagt
<210> 155
<211> 133
<212> DNA
<213> Artificial Sequence
<400> 155
cagcccggaa aggtaataca agtaagatga ttataaacaa atgctttaaa acagagtcaa 60

tgaaaccagt ctgtttgtga ggcccaaggc tccatatttt acaactcagt ctgtaaggat 120
agctatgtat ctg

## Description

### Technical field

The invention relates to medicine and veterinary science and discloses a novel method for treating oncological, virulent and somatic diseases when the main target for therapeutic action is embodied in the form of DNA which freely circulates in blood plasma (and other liquid media) and originates from tumoral and mutant cells or cells infected by bacteria, fungi or protozoan and from different microorganisms which reside in the organism thereof. Said invention also relates to novel pharmaceutical compositions and to the use thereof for treating oncological diseases and infectious states provoked by bacteria, fungi and protozoa and non-infectious somatic diseases and states produced by accumulation of somatic mutations in cells of organism. Medicinal and immunological compositions, sorption and physico-chemical engineering and the method for using them in order to treat malignant tumors and to prevent their relapses as well as for treatment of infections, atherosclerosis, diabetes and slowing the aging process. Proposed method is based on completely new principle of therapeutic action, provide strong antitumor and antimicrobial efficacy and could be used in therapy of oncological diseases, different infections and non-infectious diseases.

### Background art

Populations of cancer cells, developing in patient's organism possess extremely high level of genetic plasticity that is much higher than that in normal cells. Genetic plasticity within cancer cells populations allows them to generate in course of disease the phenotypes which are resistant to immune and morphogenic control and which has the capacity for invasion and metastasis and are resistant to antitumor therapies. It is known, that selection and clonal expansion of tumor cells comprise the basis of biological and clinical tumor progression. According to that knowledge, the modem strategy of anticancer therapy is based on principles of tumor cell clones destruction in patient organism through chemotherapy, radiotherapy, immunotherapy, surgical elimination and by use of their combinations. Fundamental particularity of all these methods is that their sole therapeutic target is a tumor cell. The long term experience from clinical application of such therapy has shown that due to high genetic placticity the most malignant cells become insensitive to the specific therapy used before malignant neoplasm could be completely destroyed by said therapy.

There is a great medical need for new antitumor drugs less toxic than existing ones or can potentiate thre efficacy of existing methods. As well it is a need for new antitumor drugs that can decrease toxicity of currently available methods whithout decreasing of their efficacy.

The circulation of DNA molecules in blood plasma of cancer patients and healthy people was known from some publications (P.Anker et al., Clinica Chimica Acta,v.313, 2001, pp.143-146; Fedorov N.A. et.al., Bull.Exp.Biol.Med.,v102,1986, pp281-283. Patent (US 5952170) disclouse the determination of DNA content in blood plasma for diagnostic and prognostic purposes in course of cancer disease. Patents (US 6465177 and US 6156504) disclouse the use of blood plasma DNA for mutation's determination in oncogenes and microsatellite gene regions, for genetic instability studying in tumors and the use of these results for diagnostic, monitoring and prognostic purposes in the course of disease.

Sugihara S. et al. (1990, 1993) studied 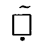 chemotrypsine and desoxiribonuclease I (DNase I) enzymes influence on autologic and heterologic adhesion of tumor cells during their metastasing. They have shown that systemic introduction of DNase I could slow metastases development. The effect they find was therapeutically insufficient. Authors make conclusion that DNase I can be used together with surgical elimination of tumor for prevention of tumor cells' hematogenic dissemination. Authors meant the influence of DNase I on tumor cells' cytoplasmatic membrane but not on destruction of free circulating DNA. Accordingly, the regime and doses that were used could not cause the decrease of circulating DNA level.

Torchilin V.P. (2001), Patent (US 5,780,033), disclouse the therapeutic use of autoantibodies that are able to bind with cytoplasmic and nuclear membranes of tumor cells and with protein-DNA complex coming from dead tumor cells. It is clear from the text of application that the disclousure relates solely to antibodies against protein antigenic determinants. In our case anti-DNA antibodies and anti-DNA abzymes are used. Moreover, therapy discloused by authors was trended against phagocytosis of nucleosomes on the surface of tumor cells and that exclude the possibility of therapeutic regimes sufficient for binding and removal of circulating blood plasma DNA.

There is practically no data concerning circulation of bacterial DNA in blood. In human organism all microorganisms exist in biofilm communities (Davey M.E. Otoole G.A. 2000. Microbial biofilms: from ecology to Molecular ganetics. Microbiol. Mol.Genet. 64:847-867). Biofilms are formed by bacterial cells, associated with extracellular matrix (Tez V.V. 1999. Formation and structure of mixed bacterial communities. APMIS, 107:645-654). In biofilm matrix we have found extracellular DNA that penetrates from living cells. Our data also indicates that bacterial DNA present in blood plasma of infected human and its quantities and composition could change subject to development of certain infections. It is also known that DNA can get into the outer environment as a result of cells death, for example in the focus of inflammation. DNA as polymer increases biofluids viscosity and that negatively influence the course of the disease, preventing elimination of toxins, pathogens and necrotic debris. It is known that-DNase (Gentech-Roch) "Pulmozyme" is used for Cystic Fibrosis treatment by inhalation. The therapeutic action is based on dilution of broncho-alveolar secret, and does not relate to breach of genetic information traffic.

There is no human or animal data, regarding the genetic composition of DNA, circulating in blood plasma. There is no data regarding blood plasma DNA analysis avoiding PCR (Polymerase Chain Reaction). The use of PCR can considerably distort the representation of DNA composition because of amplification primer's specificity. So far , the genetic analysis of plasma DNA in cancer patients were done by PSR method or blot hybridization and was directed to studying of genome changes in certain specific genome areas (for example in microsatellites and specific genes).(Sanchez-Cespedes M., et al., Ann Oncol,1998,v9(1),pp113-116; Sozzi G., et al., Clin Can Res, 1999, v5(10),pp2689-2692; Chen X.Q., et al., Nat Med,1996,v2(9),pp1033-1035).

Thus, there is no data available about genetic repertoire of DNA which circulates in blood plasma of patients with oncopathology, infections, somatic pathology and healthy people. , about the biological role of of blood plasma circulating DNA and about possible therapeutic effect of blood plasma circulating DNA destruction or inactivation in these diseases treatment.

### Disclosure of the invention

As a result of our work upon the invention we have unexpectedly found that blood plasma circulating DNA from oncological patients contain unique quantitative and qualitative repertoire of genes and regulatory genetic elements, which is strongly different from that of human genome. Circulating blood plasma DNA of oncological patients for the most part contains unique human genes, including genes that are associated with maintenance and formation of malignancy.

It was shown that circulating blood plasma DNA of patients with oncopathology participates in intercellular transport of genetic information within tumor cells' population in patient organism. Current invention disclosure methods of free blood plasma circulating DNA destruction and inactivation which lead to suppression of tumor development in host organism. The invention also disclosure new method for identification of genomic structures which are involved in tumor progression and human genome operation. This aspect of invention is connected with DNA samples isolation from blood plasma of healthy people and patients with oncological diseases, cloning and sequencing them.

It was found out that DNA from biofilm matrix secreted by different bacteria enters into the blood and biological fluids of human and animals. It was stated that the presence of extracellular DNA is one of conditions necessary for microbial infection development.

Invention includes destruction or inactivation of circulating microbial DNA as method for treatment and prophylaxis of diseases caused by them.

It was also found out that DNA circulating in blood of healthy people play role in somatic mosaicism's development. Binding, destruction or inactivation of that DNA could suppress the somatic mosaicism development. Binding, destruction or inactivation of circulating blood plasma DNA could be of therapeutic value in treatment of diseases which progression is linked with somatic mosaicism development.

One aspect of invention discloses the pharmaceutical compositions and non pharmaceutical methods of free circulating DNA destruction or inactivation in blood plasma of patients with oncological or infectious diseases.

Another invention aspect discloses treatment method of patients' with oncopathology, infections, somatic diseases and method of extension of life time, which are based on administration of pharmaceutical compounds or application of non pharmaceutical methods providing the destruction or inactivation to free circulating blood plasma DNA.

Another aspect of invention provide the way of treatment efficacy control, when said treatment is directed to destruction or inactivation of free circulating blood plasma DNA, then the monitoring of free circulating blood plasma DNA and determination of tumor-specific genetic markers or genetic microbial markers presence in blood plasma could be used for treatment efficacy control.

Methods of patients' treatment with oncopathology and infectious diseases based on pharmaceutical compounds administration or use of non pharmaceutical methods that lead to destruction or inactivation of free circulating blood plasma DNA, when such treatment is combined with use of antitumor or antimicrobial therapy were disclosed.

Genetic placticity of tumor cells , which allows them to accumulate and maintain signs that comprise "malignant phenotype", reveals itself on genetic and chromosomal levels by loss, acquiring or changing of DNA consequences, starting from single nucleoside up to whole chromosomes (Loeb K.R. et.al., Carcinogenesis, v21,2000,pp.379-385).

Source of such variability is thought to be a special *modus operandy* of tumor cells' genetic apparatus. It is characterized by increased frequency of spontaneous mutations while activity of reparative system is decreased and control system of genetic homeostasis is switched off (Schmutte C., et al., Anticancer Res., 1999, v19, pp.4665-4696).

It is thought that "mutator" phenotype of tumor cells (Loeb L.A.,Cancer Research,2001, v61,pp.3230-3239), dynamic heterogeneity within tumor cell clones (Heppner G.H. et al., International Review of Cytology, 1998, v177, pp.1-56) and numerous rounds of selection in the course of progression (Cahill D.P. et al., Trends in Cell Biology, v9, pp.M57-M60 ; Rubin H., Adv Cancer Res, 2001,v83,pp.159-207; P.Nowell, Seminars in Cancer Biology, v 12, 2002, pp.261-266) lead to selection and following expansion of the most malignant tumor clones. According to this knowledge the modem methods of malignant neoplasm's treatment are based on principle of tumor cells destruction in patient organism.

In the process of investigation we have unexpectedly found that accumulation of genetic changes that are essential for "malignant phenotype" formation in clinically advanced tumor is a result of cooperative interclonal interaction inside population of tumor cells in patients' organism, based on horizontal transfer of genetic information

Free circulating DNA in blood plasma of oncological patients is a messenger of such cooperative relations. It realizes intrapopulational interclonal transport of genes, which are responsible for "malignant phenotype" formation.

Destruction or inactivation of free blood plasma circulating DNA will lead to inability of tumor cells to maintain necessary level of genetic variability in organism and loss ability to maintain "malignant phenotype" (growth, metastasing, insensitivity to therapy). Such therapeutic intervention has its own therapeutic value as well as it increases efficacy of traditional therapeutic methods.

### Brief description of the drawings

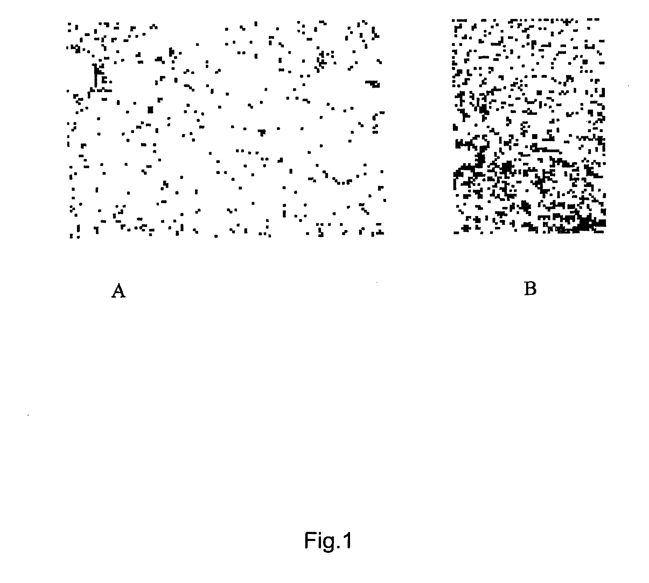

P-glycoprotein expression in murine tumors
Fig.1 Results of immunochemical staining of mice tumor's histological slices after administration of 5 day course of Doxorubicin therapy (2 mg/kg every day intravenous) and DNase I (0,5 mg/kg four times a day during 5 days).
A - Doxorubicin + DNase
B - Doxorubicin

### Preferred embodiment

Isolation of free circulating DNA from blood plasma.

Fresh blood plasma (not more than 3-4 hours after isolation) with anticoagulant (sodium citrate) addition was centrifuged on Ficoll-PlaquePlus (Amersham-Pharmacia) step at 1500g for 20 minutes at room temperature. Plasma (1/2 of all amount) was neatly isolated avoiding touching cells sediment on ficoll step and was centrifuged at 10 000 g for 30 minutes to eliminate cells and debris. Supernatant was taken away not touching sediment and up to 1% of sarcosile, up to 50 mM of tris HCl pH 7,6, up to 20 mM EDTA, up to 400 mM NaCl and equal volume of phenol - chloroform mixture 1:1 were added. Received emulsion was incubated at 65°C for 2 hours than phenol-chloroform was separated by centrifuging at 5000g during 20 minutes at room temperature. Deproteinization by phenol-chloroform method was identically repeated for three times after what water phase was processed by chloroform and after it by diethyl ether.

Organic solvents' separation was done by centrifuging at 5000g during 15 minutes. Equal part of isopropanol was added to water phase and incubated during night at 0°C.

After sedimentation nucleic acids were separated by centrifuging at 10 000g during 30 minutes. Sediment of nucleic acids was dissolved in buffer that consisted of 10 mM tris - HCl, pH 7,6, 5 mM EDTA and was inflicted on step made from chlorine cesium gradient (1M, 2.5M 5.7) in centrifuge test tube for SW60Ti rotor. DNA volume was 2 ml, volume of each CsCl step was 1 ml. Ultracentrifuging was done in L80-80 (Beckman) centrifuge for 3 hours at 250000g. DNA was isolated according to fractions from the step's surface 5.7M. Fractions were dialized during 12 hours. mM tris-HCl, pH 7,6, 1mM EDTA at 4°C will be added. DNA presence in fractions was defined by agarose electrophoresis with DNA visualization by ethidium bromide. DNA amount was spectrophotometrically estimated (Beckman DU 470) in cuvette with volume 100 mkl, using 220-320 nm spectrum. Average runout of DNA was 10-20ng according to 1 ml of plasma.

Cloning and sequencing of blood plasma DNA.

We have developed new method of DNA isolation and cloning from blood plasma, that allows to construct not amplified plasmide library of such DNA with representativeness up to million clones with average size 300-500 base pair isolated from 50 ml of blood, even taking into account significant amount of elevated liposaccharides level and non identified mixtures that troubled purification of nucleic acids. So representative analysis can be done with less amount of plasma pattern - 10-20 ml depending on pathological contaminates' presence.

Isolated according to above-mentioned method DNA was deproteinized with the use of proteinase K (Sigma) at 65°C for tightly-bound proteins elimination. After deproteinization DNA was processed by phenol-chloroform at 65°C and sedimented by 2,5 volumes of ethanol during night. After it DNA was processed by EcoRI restrictase during 3 hours or by Pfu polymerase (Stratagene) at the presence of 300 mkM of all desoxynucleothydethreephosphates for "sticky" edges elimination. Completed DNA was phosphorylated by polynucleotide kinase T4 (30U, 2 hours). Received samples/preparations were ligated in Bluescript (Stratagene), plasmid digested by EcoR1 or PvuII accordingly and dephosphorylated by alkaline phosphatase CIP (Fermentas) during 1 hour. 1 mkg of vector and 0,1-0,5 mkg of serum DNA were usually used for ligation. Ligation was done by Rapid Ligation Kit (Roche) use for 10 hours at 16°C. Volume of ligase mixture was 50 mkl. Ligated library was transformed into DH12S (Life Technologies) cells with electroporator (BioRad) use. For transformation of one library 12-20 electroporation cuvettes were used. Dilutions of the library at concentrations 10⁻⁴, 10⁻⁵ and 10⁻⁶ were plated for control on dishes with 1,5% agar and LB media, supplemented with 100mkg\ml ampicillin. In both cases library's representativeness was approximately 2-3x10⁶ clones.

Theoretically set of DNA sequences that circulate in plasma should correspond to set of genome's DNA sequences. Usually cells apoptosis is accompanied by quantitative and nonspecific DNA degradation before its exit out of the cell, so the most wide spread DNA in plasma should be repetitive elements of genome in proportion that correspond to nonspecific degradation of DNA.

Such elements are L1 repeats, satellite DNA, Alu, MER, MIR, THE repeats and some others. Quantity of unique sequences should be small in accordance to their small percent in human genome they may be not detected in cloning DNA without PCR.

Blood plasma DNA library of oncological patient with clinically advanced tumor stage.

We have constructed blood plasma DNA library of patient with diagnosed advanced stage mesothelioma. Representativeness of library was 8,5x10⁵ clones, that is a good result, taking into account rather small amount of DNA (5 □g) received after purification from non character for healthy donors liposaccharides that were in extremely high concentrations at plasma of patient.

We have got the unexpected results after analysis of 96 clones with length from 300 up to 1000 base pairs. (It is necessary to mention that only one clone was not identified as human DNA. For all others correspondent information from HumanGenBank that identifies DNA of these clones as human DNA was received.) As mentioned above, according to data from literature it is logically to assume that there will be a lot of higly repeatitive elements in DNA samples.

But at least 55 out of 96 clones presented unique sequences of human DNA. Taking into account real ratio of repeatitive and unique elements of human genome (95% to 5%) it is obvious that blood plasma DNA repertoire of this patient differs a lot from human genomic DNA repertoire. In this sample an abrupt enrichment by unique DNA sequences is observed.

For 15 out of 55 unique DNA fragments that were identified during sequencing of 96 clones from the library of blood plasma DNA, functions or product of correspondent gene were identified. Tables 1-15 present list of these sequences and information about their participation in formation and maintenance of "malignant phenotype".

**Table 1**

| Clone | Product | Participation in oncogenesis | Source |
|---|---|---|---|
| Clone 24 | Member of G protein - coupled receptor family | Playing major role in cancer cell signalling. Linked with cell transformation, supression of apoptosis, hormone independence and metastasis | Steeg P.S. ,Nat Rew Cancer,2003,v.3,pp .55-63. Raj G.V. ,J Urology,2002, v.167, pp.1458-1463. Hoff A.O., Neoplasia,1999 v.1,pp.485-491. |

**Table 2**

| Clone | Product | Participation in oncogenesis | Source |
|---|---|---|---|
| Clone 43 | Snf2-coupled CBP activator protein (SCRAP) | Transription activator. Family members linked with synovial sarcoma and leukaemia development | Thaete C., Hum Mol Genet,1999,v.8,pp. 585-91. Monroy M,A.,J Biol Chem,.2001,v.276, pp.40721-40726 Lee D.W., Cancer Res., 2000, v.60,pp.3612-3622. |

**Table 3**

| Clone | Product | Participation in oncogenesis | Source |
|---|---|---|---|
| Clone 51 | SRY-box containing gene | Transcription modulator . Expressed during embryogenesis. Family members linked with medulloblastomas, gonadal tumors, highly metastatic melanoma. | Graham J.D., J Mol endocrinol, 1999,v.22,pp.295-304. Lee C.J.,J Neurooncol, 2002,v.57,pp.201-214. Uehara S., Cancer Genet Cytogenet,1999,v. 113.,pp.78-84. Tani M.,Genomics, 1997,v.39,pp.30-37 |

**Table 4**

| Clone | Product | Participation in oncogenesis | Source |
|---|---|---|---|
| Clone 72 | Protein- tyrosine kinase | Family members playing major role in cancer. Some PTK are cellular specific oncogenes products. | Hunter T., Philos Trans R Soc Lond B Biol Sci, 1998,v.353.,pp.583 -605. Scheijen B.,Oncogene, 2002,v.21.,pp.3314 -3333. |

**Table 5**

| Clone | Product | Participation in oncogenesis | Source |
|---|---|---|---|
| Clone 83 | Fibroblast activation protein alpha; cell surface serine protease | Family members playing role in cancer invasion and metastasis. The product is active in cancer stroma and different carcinomas. | Chen W.T, Enzyme Protein, 1996,v.49., pp.59-71. Scanlan M.J.,Proc Nat Acad Sci USA, 1994, v.91, pp.5657-5661. Mathew S., Genomics, 1995,v.25,pp.335-337. |

**Table 6**

| Clone | Product | Participation in oncogenesis | Source |
|---|---|---|---|
| Clone 86 | Brain testican | Proteoglycan with unknown function. Linked with neoplastic phenotype of embryonal rhabdomyosarcoma cells. | Genini M., Int J Cancer, 1996,v.66,pp.571-577. |

**Table 7**

| Clone | Product | Participation in oncogenesis | Source |
|---|---|---|---|
| Clone 152 | KRAB domain, Zn-finger proteins | Family members are known as transcription repressors. Linked with early embryogenesis, neuroblastoma, Ewing sarcoma, Tcell lymphoma, in progression and chemoresistance in lung cancer. | Oguri T.,Gene,1998,v.22 2,pp.61-67 Gou D.M.,Biochim Biophys Acta, 2001,v.1518,pp.30 6-310 Margolin J.F.,Proc Nat Acad Sci USA, 1994,v/91,pp.4509 -4513. Bellefroid E.J.,EMBO J, 1993,v.12,pp.1363-1374. Gonzales-Lamuno D.,Pediatr Pathol Mol Med, 2002,v.21,pp.531-540. Marilee J.W.,Gene, 1994,v.152,pp.227 -232. |

**Table 8**

| Clone | Product | Participation in oncogenesis | Source |
|---|---|---|---|
| Clone 190 | Antigen linked with melanoma | Antigen recognized by autologous tumor infiltrating lymphocytes. | J.Immunol.166(4), 2871-2877,2001 |

**Table 9**

| Clone | Product | Participation in oncogenesis | Source |
|---|---|---|---|
| Clone 167 | N-cadherin | Cell adhesion molecule with major role in cancer growth, invasion and metastasis. | Hazan R.B.,J Cell Biol, 2000,v.148,pp.779 -790. Li G.,Cancer Res, 2001,v.61,pp.3819 -3825. Tran N.L.,J Biol Chem, 2002,v.277,pp.329 05-32914. |

**Table 10**

| Clone | Product | Participation in oncogenesis | Source |
|---|---|---|---|
| Clone 197 | FAF1: Fas associated factor 1 | Phosphoprotein known to be the proapoptosis factor. | Jensen H.H.,Int J Biochem Cell Biol, 2001,v.33,pp.577-589. Ryu S.W.,Biochem Biophys Res Commun, |

**Table 11**

| Clone | Product | Participation in oncogenesis | Source |
|---|---|---|---|
| Clone 114 | Interleukin 7 | Proposed as essential paracrine \ autocrine growth factor for variety of cancers. | Trinder P., Int J Oncol, 1999,v.14,pp.23-31. Cosenza L.,Cell Signalling, 2002,v. 14,pp.317-325. |

**Table 12**

| Clone | Product | Participation in oncogenesis | Source |
|---|---|---|---|
| Clone 208 | DEAD Box RNA helicase - like protein | Family members involved to RNA methabolism. Linked to exponential cell growth in cancer. | Iggo R.D.,Mol Cell Biol, 1991,v.11,pp.1326 -1333. Causevic M.,Oncogene, 2001,v.20,pp.7734 -7743. |

**Table 13**

| Clone | Product | Participation in oncogenesis | Source |
|---|---|---|---|
| Clone 97 | Lipin 1 | One of tumor cells' response regulators on cytotoxic compounds. | Brachat A. et.al., Oncogene, 2002,v.21,pp.8361 -8371 |

**Table 14**

| Clone | Product | Participation in oncogenesis | Source |
|---|---|---|---|
| Clone 121 | Dynein | Takes part in transport of p53 protein, is hypersecreted at cancer of prostate and hepatocellular cancer. | Bull J.H.,et.al.,Br J Cancer,2001,v.84,p p.1512-1519. Giannakakou P., et.al., Nat Cell Biol, 2000,v.2,pp.709-717 Jiang J.,et.al.,Gene, 2001,v.281,pp.103 -113. |

**Table 15**

| Clone | Product | Participation in oncogenesis | Source |
|---|---|---|---|
| Clone 178 | Ramp protein | Linked with human embrional carcinoma cells' development. | Cheung W.M., et.al., J Biol Chem,2001, v.276,pp.17083-17091 |

In this way 14 out of 15 sequences with identified function or protein that encodes different products (protein kinases, growth factors, proteinases, adhesive molecules and regulatory nuclear proteins) are described in literature as related to "malignant phenotype" formation and maintenance. Only product of 197^{th} clone identified as pro-apoptotic factor is not clearly linked with malignant progression. Though there is data concerning relationship between high apoptotic activity of tumor with its progression (Nishimura R., et al., J Surg Onco1,1999,v.71,pp.226-234) and possible role of apoptotic inductors in formation and maintenance of immunosupression in malignant growth (O'Connel J., et al., Dis Esophagus,1999,v.12,pp.83-89).

The most significant presence from repeatitive elements in this material was alpha-satellite DNA (30 clones). It is possible to say that that alpha-satellite DNA was the only highly repeated element from human genome, which behaves exactly as repeat in this material. The rest of highly repeatitive elements were presented in material as one or several clones (L1 variant and MLT26), or were not found among patterns (MER, Alu, THE, MIR, □satellite). Based on today's knowledge one can assume that plasma blood composition for the most part should repeat composition of genome DNA, so listed repeats should be represented in the major part of clones while unique and moderately repeated consequences in analysis of such a small number of clones should not be recognized at all. Received result clearly indicates on special way of plasma DNA formation in oncological patients. Another unexpected result is that of finding in the material of two new moderately repeated sequences - duplicones, that were recently unknown, also support the evidence of special way of plasma DNA formation in patients with malignant tumor. For the first time duplicones were found in human genome less then two years ago. Known duplicones (Eichler E.E.,et al.,Genome Res,1998,v8,pp.791-808; Ji Y., et al.,Genome Res,2000,v.10,pp.597-610; Pujana M.A.,et al.,Genome Res,2001,v.11,pp.98-111) are extensive regions of DNA that were multiplied for several times in the frame of one chromosome (unlike other repeats that are randomly allocated in genome).Duplicones' formation and expansion is connected with different genetic syndromes (for example Prader-Willi/Angelmane syndrome), with multigenic families' evolution such as MHS (Shiina T., et al.,Proc Nat Acad Sci USA,1999,v.96,pp.13282-13287) and with chromosome instability in tumors.

It is necessary to mention that analysis of clones received from blood plasma DNA of patient has given us unexpected results.

Blood plasma DNA of oncological patient is highly enriched with unique genes. 55 out of 96 analyzed clones contain fragments of genome's unique sequences. 14 out of 15 sequences with identified in functions refer to process of tumor progression and maintenance of "malignant phenotype".

Strong impoverishment of the most wide spread human repeat such as MER, Alu, THE, MIR, 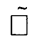 satellites is found in plasma DNA material.

Finding of two consequences with previously unknown duplicones' characteristics indicates on duplicones' representativeness in such DNA samples.

DNA library of healthy donor's blood plasma.

For method's value proof of blood plasma DNA cloning and sequencing for identification of genome's unique genetic consequences we have constructed DNA library of healthy donor's blood plasma. It is known that plasma of clinically healthy people also contains DNA but in significantly less amount than plasma of oncological patients (Shapiro B., et al., Cancer,1983,v.51,pp.2116-2120).

Representativeness of library was near 8x10⁵ clones. We have got interesting result after analysis of 70 clones with length from 300 up to 1000 base pairs. We found out that 58 out of 70 analyzed clones are unique DNA sequences of human genome. After searching the HumanGenBank, we identified the function or product of correspondent gene for 14 out of 58 unique DNA fragments.

Only 12 clones contained fragments of repetitive sequences, herewith without of alpha-satellite DNA dominance.

So, it was unexpectedly found out that blood plasma DNA of healthy people and oncological patients for the most part contain unique fragments of human genome. In the case of oncological pathology unique sequences of blood plasma DNA correspond to genes which products take part in the formation and maintenance of tumor cell's "malignant phenotype".

Basing on this unexpected discovery we have suggested that DNA circulating in patient's blood can be messenger of horizontal genetic information transfer during the course of oncological diseases, assisting to accumulation and spreading of genes that are necessary for "malignant phenotype" formation and maintenance whithin population of tumor cells.

Somatic mosaicism is a condition that is a result of genetically non-identical somatic cells' presence in organism. Modem vision presents that many non tumor and noninfectious (so called somatic) human diseases (for example atherosclerosis, diabetes, nonspecific chronic lung diseases and so on), including aging process, are connected with appearance and spreading (expansion) in the process of individual development of somatic cells' clones that have mutant genes. (Youssoufian H., et al., Nature Rew. Genet., 2002,v.3,pp.748-758; J.Vijg, Mutation Res.,2000,v.447,pp.117-135; R.Erickson, Mutation Res.,2003,v.543,pp.125-136; Andreassi M.,Mutation Res.,2003,v.543,pp.67-87;Anderson G., et al., Trends in Pharmacological Sci.,2003.,v.24,pp.71-76).

Bright example of such process is progression of mitochondrial heteroplasmia (expansion of mutant mitochondrial DNA) at different diseases and in the aging process (E.Jazin et al., Proc Nat Acad Sci USA, 1996, v.93,pp.12382-12387; Michikawa Y. et al., Science, 1999, v.286, pp.774-779;Calloway C. et al., Am J Hum Gen,2000,v.66,pp.1384-1397).

There are two alternative models of somatic mosaicism's appearance. The first is appearance of somatic mosaicism as a result of numerous "de novo" mutations in polyclonal cellular pool. The second model is clonal expansion of mutant cells' clone (Khrapko K., et al., Muation Res.,2003,v.522,pp.13-19).

In the process of work above the invention we have found that DNA circulating in the blood of healthy people play significant role in somatic mosaicism's development and its binding, destruction or inactivation inhibits development of somatic mosaicism. Binding, destruction or inactivation of circulating in blood plasma DNA provides treatment effect at diseases which appearance is connected with somatic mosaicism's development.

Examples that are mentioned later indicates role of circulating in blood of oncological patients DNA in the development of tumor's resistance to chemotherapy, development of metastasing process, in sepsis development and in some other pathological conditions. High therapeutic effect of blood plasma DNA's binding, destruction or inactivation is found.

### Role of free circulating DNA in tumor progression

Materials and methods.

Bovine pancreatic DNase I (Fermentas) and human recombinant DNase I (Dornase; Gentech) were used. DNase I solution for injections were prepared by dissolving of DNase matrix solution in sterile phosphate buffer directly before administration. Cyclophosphamide and Doxorubicin were dissolved according to instruction.

In series of in vitro experiments we have not seen any supressive effect of DNase I on tumor cells' growth (DNase I concentration was up to 100 mkg/ml).

Blood plasma DNA of tumor-bearing mice was received according to technique described above. Highly metastatic and low metastatic stains of mice' Luice lung carcinoma and Erlich carcinoma were used. Cells were cultivated in RPMI-1640 media with adding of 10% embryonic calf serum, 1% penicillin - streptomycin in 5% carbon dioxide atmosphere. For tumor induction in mice, cells were cultivated until the monolayer , than were detached with trypsin-EDTA solution . Cells were washed trice by centrifuging in phosphate buffer and were resuspensed until their concentration have become 0,5x10⁷/ml. Viability was defined by inclusion of methylene blue in hemocytometer. Suspensions that contained 95% of viable cells were used for administration to animals.

Mice of C57B1 line and white not thoroughbred mice from "Rappolovo" animal house were used. Weight of animals was 24-26 grams. Animals were contained in cages, for 6-7 mice per one cage on a standard diet without water restriction. LLC cells in dose of 5x10⁵ in 100 mkl of phosphate buffer were injected in soft tissues of thigh. Erlich tumor was subcutaneously replanted on the right side by injection of 0,2 ml 10% cells' suspension in isotonic sodium chloride solution.

In some experiments DNA presence in blood plasma of mice was studied.

DNA was isolated by method that is mentioned above. DNA amount was spectrophotometrically measured.

The invention is illustrated by following examples.

### Example 1.

### Influence of DNase I administrated twice a day on Erlich tumor growth at mice.

Group 1-10 mice transplanted with Erlich carcinoma (control).
Group 2-10 mice transplanted with Erlich carcinoma. Mice were intraperitoneally injected with DNase at 1 mg/kg dose in 200 ml of phosphate buffer twice a day starting from 3 up to 7 day after tumor transplantation.
Group 3-10 mice transplanted with Erlich carcinoma. Mice were intraperitoneally injected with DNase at 2 mg/kg dose in 200 ml of phosphate buffer twice a day starting from 3 up to 7 day after tumor transplantation.
   - Results of experiments were estimated according to tumor's growth inhibition (TGI) that were expressed in per cents to control data received at last day of DNase injection with standard formula using.

Tumor's size on the 7 day after transplantation.

| Group | Tumor's volume | TGI% | P |
|---|---|---|---|
| 1 | 86+/-12 | - | - |
| 2 | 3 3+/-6 | 61% | p<0,001 |
| 3 | 34+/-7 | 60% | p<0,001 |

The data obtained indicates that tumor's growth is significantly inhibited by DNase injection.

### Example2

### Inhibition of Erlich tumor growth by use of different schemes of DNase administration.

In our experiments DNA circulating in blood plasma of patients was the therapeutic target of DNase. Prolonged presence of DNase in blood plasma at catalytically effective concentrations is essential for provision of maximal therapeutic effect. According to that DNase myltiply administration should provide better therapeutic effect than the same total daily dose, administrated just as only two daily injections.
Group 1 - 10 mice transplanted with Erlich carcinoma (control).
Group 2-10 mice transplanted with Erlich carcinoma. Mice were intraperitoneally injected with DNase at 1 mg/kg dose in 200 ml of phosphate buffer twice a day starting from 3 up to 7 day after tumor transplantation.
Group 3 - 10 mice transplanted with Erlich carcinoma. Mice were intraperitoneally injected with DNase at 0,5 mg/kg dose in 200 ml of phosphate buffer four times a day starting from 3 up to 7 day after tumor transplantation.
• Results of experiments were estimated according to tumor's growth inhibition (TGI) that were expressed in per cents to control data received at last day of DNase injection with standard formula using
Tumor's size on the 7 day after transplantation.

| Group | Tumor's volume | T% | P |
|---|---|---|---|
| 1 | 98+/-14 | - | - |
| 3 | 23+/-6 | 76% | P<0,001 |
| 2 | 32+/-6 | 67% | P<0,001 |

Received data indicates that tumor's growth is more inhibited by fractional (four times a day) administration of DNase at the same total daily dose than twice a day administration. Blood plasma DNA concentration of mice from 3 group after end of treatment course was 38,5 ng/ml, mice from control group had 104,8 ng/ml and mice from the 2 group - 55,1 ng/ml. Blood plasma DNA concentration of healthy mice was 12,0 ng/ml (p<0,01). So, multiply daily administrations of DNase lead to more significant decrease of blood plasma DNA level and greater tumor's growth inhibition in comparison with twice a day administration of the same total dose.

### Example 3

### Combined use of DNase and antitumor Doxorubicin compound.

Group 1-10 mice transplanted with Erlich carcinoma (control).
Group 2-10 mice replanted with Erlich carcinoma. Mice were intravenously injected with Doxorubicin at 2 mg/kg dose once a day starting from 3 up to 7 day after tumor transplantation.
Group 3 - 10 mice replanted with Erlich carcinoma. Mice were intravenously injected with Doxorubicin at 2 mg/kg dose once a day starting from 3 up to 7 day after tumor transplantation. As well mice were intraperitoneally injected with DNase at 0,5 mg/kg dose in 200 ml of phosphate buffer four times a day starting from 3 up to 7 day after tumor transplantation.
Group 4-10 mice replanted with Erlich carcinoma. Mice were intraperitoneally injected with DNase at 0,5 mg/kg dose in 200 ml of phosphate buffer four times a day starting from 3 up to 7 day after tumor transplantation.
• Results of experiments were estimated according to tumor's growth inhibition (TGI) that were expressed in per cents to control data received at last day of DNase injection with standard formula using.
Tumor's size on the 7^{th} day after transplantation.

| Group | Tumor's volume | T% | P |
|---|---|---|---|
| 1 | 98+/-14 | - | - |
| 2 | 57+/-10 | 42 | P<0,05 |
| 4 | 20+/-6 | 78 | P<0,01 |
| 3 | 0 | 100% | |

Received data indicates that tumor's growth is less inhibited by Doxorubicin than by DNase. Combined use of Doxorubicin and DNase present synergism in their action leading to complete inhibition of tumor's growth (tumors were not found in any experimental animals).

### Example 4

### Influence of DNase on bacterial biofilm formation.

For estimation of possible mechanisms of DNase action on bacteria' interactions with host's organism we have estimated its influence on biofilm formation.

Experiments were performed on biofilm models, received at laboratory cultivation on the surface of glass. Non-relative Gram-positive (Staphylococcus aureus VT-209) and Gram-negative (Escherichia coli ATCC25922) bacteria were used.

Bacteria were sowed at 10⁹ bacteria/ml concentrations and were incubated at 37°C during 72 hours on synthetic M9 media that contains additives necessary for used bacteria strains. DNase was added in amount of 100 mkg/ml right after bacteria' inoculation. Identical volume of phosphate buffer was added to control. Selective vials were taken for analysis every 24 hours.

Glasses were washed by PBS buffer, fixed, stained with methylene blue and studied with the use of light microscopy.

As a result it was found out that in DNase presence normal biofilm was not formed. Only adhesion of single microorganisms to the glass that have not lead to the formation of microcolonies and biofilms was observed.

Control sowings from vials for definition of colony-forming units(**CFU**) have shown that at DNase presence there is no death of cells leading to decrease of **CFU**'s numbers in comparison with controls. Received data indicates that DNase do not cause death of Staphylococci and Escherichia, but prevent their cooperative behavior that leads to biofilm formation.

### Example 5

### DNase' use for treatment of the experimental sepsis.

White not thoroughbred mice were used for experiment. Animals' weight was 24-26 grams. Pathogenic strain of Staphylococcus aureus VT-2003R at 1x10¹⁰ bacteria/animal dose was inoculated to retroorbital sinus of animals. DNase was intraperitoneally administred. Isotonic solution of sodium chloride or Penicillin was administrated to control groups. Each experimental group contained 10 mice. Intraperitoneal administration of compound (DNAase) four times a day at 500 mkg/kg dose continued for 1-3 days after infection. Penicillin was intramusculary administrated. Efficiency of action was estimated by number of animals that stayed alive after the death of the last one in control group. All animals have died to the third day of the experiment in control group. Six animals stayed alive to the third day of the experiment in the group that administrated DNase. Protection was 67%. Received data specify an opportunity and efficiency of DNase use for infections' treatment.

### Example 6

### Level of free circulating plasma DNA at oncological patients and healthy adults.

Blood plasma DNA of oncological patients and blood plasma DNA of healthy donors differs not only by its genetic repertoire but also by its amount circulating in blood plasma. Table below presents data of blood plasma DNA contents at 10 oncological patients and at 10 healthy volunteers. DNA isolation from plasma and determination of its amount was done according to protocol mentioned above.

| Patient | Sex, age | Tumor, stage | DNA contents; ng\ml |
|---|---|---|---|
| 1 | M, 67 | Lung carcinoma T2N2MO | 123 |
| 2 | F,37 | Breast cancer T2N0M0 | 78 |
| 3 | F,53 | Gaster carcinoma T3N2M1 | 90 |
| 4 | M,54 | Cancer of large intestine T2N2M2 | 340 |
| 5 | M,64 | Cancer of large intestine T2N1M0 | 182 |
| 6 | M,56 | Lung carcinoma T3N2M1 | 99 |
| 7 | F,49 | Cancer of large intestine T2N1M0 | 75 |
| 8 | M,65 | Gaster carcinoma T3N1M0 | 120 |
| 9 | M,36 | Osteogenic sarcoma T3N1M2 | 243 |
| 10 | F,50 | Breast cancer T3N1MO | 165 |
| 11 | M,24 | Volunteer | 10 |
| 12 | M,32 | Volunteer | 27 |
| 13 | F,21 | Volunteer | 45 |
| 14 | F,19 | Volunteer | 7 |
| 15 | F,21 | Volunteer | 13 |
| 16 | F,23 | Volunteer | 89 |
| 17 | M,28 | Volunteer | 11 |
| 18 | F,32 | Volunteer | 15 |
| 19 | F,25 | Volunteer | 17 |
| 20 | M,38 | Volunteer | 5 |

As it is clear from the table the level of volunteers' blood plasma DNA is considerably lower than in blood of patients with different malignant neoplasms.

### Example 7

### DNA clones' sequences, received from free circulating blood plasma DNA of patient with malignant mesothelioma.

Clone 1
   Duplicon, chromosome 15 and Y
   Sequence Nº1.
Clone 3
   Unique,chromosome 2.
   Sequence Nº2
Clone 8
   MLT2B repeat
   Sequence Nº3
Clone 9
   Centromeric satellite DNA
   Sequence Nº4
Clone 10
   MLT2B repeat
   Sequence Nº5
Clone 20
   L1MC4-like (LINE-element)
   Sequence Nº6
Clone 15
   Alpha-satellite DNA
   Sequence Nº7
Clones 18, 21
   Alpha-satellite DNA
   Sequence Nº8
Clone 24
   Unique, family of G protein-bound proteins, chromosome 6.
   Sequence Nº9
Clone 25
   Unique, chromosome 3.
   Sequence Nº10
Clone 26
   SatB1/Vimentin/nuclear matrix binding DNA
   Sequence Nº11
   Sequence 33
   Duplicon specific to the chromosome 10
   Sequence Nº12
Clone 32
   alpha-satellite DNA
Clone 35
   LTR repeat
   Sequence Nº13
Clone 36
   Unique, chromosome 18
   Sequence Nº14
Clone 37
   Unique, chromosome 4
   Sequence Nº15
Clone 41
   Sequence Nº16
Clone 43
   Snf2-related CBP activator protein (SCRAP)
   Unique, chromosome 16
   Sequence Nº17
Clone 45
   Unique, chromosome 3
   Sequence Nº18
Clone 47
   Alpha-satellite DNA
Clone 51
   SRY-box containing gene.
   Sequence Nº19
Clone 52
   Repeat
   Sequence Nº20
Clone 53, 55
   Alpha-satellite DNA
   Sequence Nº21
Clone 56
   Centromeric repeat
   Sequence Nº22
Clone 60
   Gene repeated on several chromosomes, contains MER5A repeat.
   Sequence Nº23
Clone 62
   Repeat
   Sequence Nº24
Clone 65
   Unique, chromosome 2
   Sequence Nº25
Clone 71
   Unique, chromosome 2
   Sequence Nº26
Clone 72
   Unique, chromosome 8
   Sequence Nº27
Clone 73
   Unique
   Sequence Nº28,
Clone 78
   Transposon Tigger fragment
   Sequence Nº29
Clone 81
   Sequence Nº30
   Repeat (LINE)
Clone 82
   Unique, chromosome 1
   Sequence Nº31
Clone 83
   Unique, Fibroblast activation protein alpha; cell surface serine protease
   Chromosome 2
   Sequence Nº32
Clone 79
   Alpha-satellite DNA
Clone 86
   Unique, gene highly similar to brain testican , chromosome 4.
   Sequence Nº33
Clone 90
   Unique, chromosome X
   Sequence Nº34
Clone 93
   Unique, chromosome 9
   Sequence Nº35
Clones 89 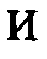 92
   Alpha-satellite DNA
Clone 96
   Fragment LINE.
   Sequence Nº36
Clone 97
   Chromosome 2 unique, Lipin
Clone 98
   Unique, chromosome X
   Sequence Nº38
Clone 102
   Chromosome 17 unique
   Sequence Nº39
Clone 99
   Alpha-sattelite DNA
Clone 105
   Unique, chromosome 13
   Sequence Nº40
Clone N106
   Chromosome 9 unique
   Sequence Nº41
Clone 107
   Unique, chromosome 8
   Sequence Nº42
Clone N111
   Unique, chromosome 12
   Sequence Nº43
Clone N 112
   Chromosome 5 unique
   Sequence Nº44
Clone 114
   Chromosome 8 unique; Interleukin 7
   Sequence Nº45
Clone 116
   Chromosome 1 unique
   Sequence Nº46
Clone 121
   Chromosome 5 unique; Dynein
   Sequence Nº47
Clone 115; 119;120
   Alpha-sattelite DNA
   Clone 125
Chromosome 9 unique
   Sequence Nº48
Clone 127
   Unique chromosome 20
   Sequence Nº49
Clone 130
   Unique, chromosome is not determined.
   Sequence Nº50
Clone124
   SatB1/Vimentin/nuclear matrix binding DNA
Clone 133
   Alpha-satellite DNA
Clone 137
   MLT1A2 repeat
   Sequence Nº51
Clone 140
   Unique, chromosome 2 ;zinc finger protein, subfamily 1A
   Sequence Nº52
Clone 141
   Chromosome 2 unique
   Sequence Nº53
Clone 143
   Fragment of Alu-repeat
   Sequence Nº54
Clone 144
   Chromosome 2 unique
   Sequence Nº55
Clone 146
   Chromosome 4 unique
   Sequence Nº56
Clone 139 and 142
   Alpha-sattelite DNA
Clone 148
   Repeat (chromosomes 1,2 and 4)
   Sequence Nº57
Clone 152
   Unique, chromosome 16;KRAB-Domain, zinc finger protein
   Sequence Nº58
Clone 154
   Chromosome 9 unique
   Sequence Nº59
Clone 161
   Fragment LINE
   Sequence Nº60
Clone 151
   Chromosome 5 unique
   Sequence Nº61
Clone 150
   Chromosome 1 unique
   Sequence Nº62
Clone 153
   Chromosome 11 unique
   Sequence Nº63
Clone159
   Chromosome 6 unique
   Sequence Nº64
Clone 163
   Alpha satellite DNA
   Sequence Nº65
Clone 166
   Chromosome 12 unique
   Sequence Nº66
Clone 167
   Unique , chromosome 18, CDH2; cadherin 2, type 1, N-cadherin
   Sequence Nº67
Clones 169, 170
   Chromosome 18 unique
   Sequence Nº68
Clone 178
   Unique chromosome 1; RAMP: RA-regulated nuclear matrix-associated protein
   Sequence Nº69
Clone 180
   Unique, chromosome 20
   Sequence Nº70
Clone 181
   Unique chromosome 18
   Sequence Nº71
Clone 185
   Alpha-satellite DNA
   Sequence Nº72
Clone 187
   Mer repeat
   Sequence Nº73
Clone 188
   HSATII repeat
   Sequence Nº74
Clone 189
   Chromosome 9 unique
   Sequence Nº75
Clone 190
   Chromosome 1 unique; melanoma antigen recognized by T cells 2
   Sequence Nº76
Clone 195
   Chromosome 10 unique
   Sequence Nº77
Clone 196
   Chromosome X unique
   Sequence Nº78
Clone 197
   Chromosome 1 unique, FAF 1: Fas (TNFRSF6) associated factor 1
   Sequence Nº79
Clone 200
   Chromosome 8 unique
   Sequence Nº80
Clone 202
   Unique chromosome 13
   Sequence Nº81
Clone 205
   Alpha satellite DNA
   Sequence Nº82
Clone 206
   Repeat
   Sequence Nº83
Clone 208
   Unique chromosome 8;Human DEAD box RNA helicase-like protein
   Sequence Nº84

### Example 8

### DNA clones sequences received from free circulating blood plasma DNA of healthy donor.

Clone 1
   Chromosome 5 unique
   Sequence Nº85
Clone 9
   Unique chromosome 21
   Sequence Nº86
Clone 7
   Unique chromosome 3
   Sequence Nº87
Clone 8
   Chromosome 4 unique
   Sequence Nº88
Clone 10
   18 S RNA gene
   Sequence Nº89
Clone 11
   Alu repeat
   Sequence Nº90
Clone 13
   Unique chromosome 3
   Sequence Nº91
Clone 15
   Unique chromosome 1
   Sequence Nº92
Clone 16
   Unique chromosome 3, neutral endopeptidase
   Sequence Nº93
Clone 17
   Chromosome 8 unique
   Sequence Nº94
Clone 18
   Chromosome 1 unique
   Sequence Nº95
Clone 21
   Unique chromosome 19;Zinc Finger protein
   Sequence Nº96
Clone 22
   Unique chromosome 18
   Sequence Nº97
Clone 23
   Unique chromosome 7, muskelin 1
   Sequence Nº98
Clone 25
   Unique chromosome 11
   Sequence Nº99
Clone 27
   Repeat
   Sequence Nº100
Clone 29
   Unique chromosome 6
   Sequence Nº101
Clone 30
   Unique chromosome 14
   Sequence Nº102
Clone 31
   Unique chromosome 17
   Sequence Nº103
Clone 32
   MER4B repeat
   Sequence Nº 104
Clone33
   Chromosome 1 unique
   Sequence Nº105
Clone 34
   Unique chromosome 2
   Sequence Nº106
Clone 35
   Repeat
   Sequence Nº107
Clone 36
   Chromosome 1 unique
   Sequence Nº108
Clone 37
   HERVH repeat
   Sequence Nº109
Clone 41
   Chromosome X unique
   Sequence Nº110
Clone 42
   Chromosome 6 unique
   Sequence Nº111
Clone 43
   Unique chromosome 22; KREMEN1
   Sequence Nº112
Clone 44
   Unique chromosome 14
   Sequence Nº113
Clone 45
   Unique
   Sequence Nº114
Clone 46
   Chromosome 20 unique
   Sequence Nº115
Clone 47
   Nf-kappaB
   Sequence Nº116
Clone 38
   Unique chromosome 16
   Sequence Nº117
Clone 48
   Chromosome 6 unique
   Sequence Nº118
Clone 53
   Unique
   Sequence Nº119
Clone 51
   Chromosome 5 unique
   Sequence Nº120
Clone 59
   Unique chromosome 4, NFKB 1: nuclear factor of kappa light polypeptide gene enhancer
   Sequence Nº121
Clone 61
   Repeat
   Sequence Nº122
Clone 62
   L 1 repeat
   Sequence Nº123
Clone 64
   Duplicon chromosome 7
   Sequence Nº124
Clone 65
   Ribosomal DNA
   Sequence Nº125
Clone 66
   Rbosomal DNA
   Sequence Nº126
Clone 75
   Repeat
   Sequence Nº127
Clone 76
   Chromosome 4 unique
   Sequence Nº128
Clone 83
   Chromosome 4 unique
   Sequence Nº129
Clone 85
   Unique chromosome 2; phospholipase C, epsilon
   Sequence Nº130
Clone 87
   L1PA3 repeat
   Sequence Nº131
Clone 86
   Unique chromosome 5; CRTL1: cartilage linking protein 1
   Sequence Nº132
Clone 89
   Alu repeat
   Sequence Nº133 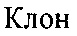 92
   Unique chromosome 6
   Sequence Nº134
Clone 100
   Unique, chromosome 6
   Sequence Nº135
Clone 105
   AluSx repeat
   Sequence Nº136
Clone 111
   Alphoid repetitive DNA
   Sequence Nº137
Clone 112
   Chromosome 9 unique
   Sequence Nº138
Clone 113
   Chromosome 22 unique
   Sequence Nº139
Clone 114
   AluSx repeat
   Sequence Nº140
Clone 116
   Unique chromosome 9; 17kD fetal brain protein
   Sequence Nº141
Clone 123
   Unique chromosome 5
   Sequence Nº142
Clone 124
   Unique chromosome 13
   Sequence Nº143
Clone 126
   Unique chromosome 8
   Sequence Nº144
Clone 130
   Unique chromosome 1
   Sequence Nº145
Clone 131
   Unique chromosome 4
   Sequence Nº146
Clone136
   Unique chromosome 8
   Sequence Nº147
Clone 141
   Unique chromosome 2
   Sequence Nº148
Clone 146
   Unique chromosome 16
   Sequence Nº149
Clone 147
   Unique chromosome 5; nicotinamide nucleotide transhydrogenase
   Sequence Nº150
Clone 149
   Unique chromosome 9
   Sequence Nº151
Clone 151
   Unique chromosome 16
   Sequence Nº152
Clone 152
   Unique chromosome 6, BAI3: brain-specific angiogenesis inhibitor 3
   Sequence Nº153
Clone 153
   Unique chromosome 9, GAD2: glutamate decarboxylase 2
   Sequence Nº154
Clone 155
   Unique chromosome 9
   Sequence Nº155

### Example 9

### Sensitivity of circulating blood DNA to DNase action.

DNA from fresh blood serum (mixture of blood received from 5 volunteers ) was isolated by standard phenol-chloroform method.

Sediment of nucleic acids was washed by 70% ethanol and dissolved in tris-EDTA buffer. Spectrophotometry with wave's length 260 nm (SF-46) was used for quantification of DNA. DNA was researched by electrophoresis in 0,8% polyacrylamide gel with ethidium bromide staining. One part of received DNA was processed by DNase I for 3 hours at 37°C. After electrophoretic separation of processed and not processed DNA it was found out that:
After electrophoresis non-processed DNA constitute one compact band. This factor indicates on relatively identical size of DNA circulating in blood. Said band disappeared completely after DNAse processing. Received data indicates that DNA circulating in serum is sensitive to DNases use and can be destroyed by their action.

### Example 10

### Results of the experiment of influence of polyclonal serum containing anti-DNA antibodies' on life span of Erlich carcinoma bearing mice.

DNA circulating in blood plasma of oncological patients can be destroyed or inactivated not only by DNA - digesting enzymes (for example by DNase), but also by other methods.

Anti-DNA antibodies were isolated from blood of patients with lupus erythematosus by Shuster A.M. method (Shuster A.M. et.al.,Science,v.256,1992,pp.665-667). Such anti-DNA antibodies were capable not only to bind DNA but also to hydrolyze it.
Group 1-7 mice transplanted with Erlich carcinoma (control).
Group 2-6 mice transplanted withy Erlich carcinoma. Mice were injected intravenously with fraction of human anti-DNA antibodies (Ig G) at 200 mkg dose on the third day after tumor transplantation.
Group 3-6 mice transplanted withy Erlich carcinoma. Mice were injected intravenously with fraction of human non specific immunoglobulin (Ig G) at 200 mkg dose on the third day after tumor transplantation.

Effect was estimated according to tumor's growth inhibition on the 7^{th} day after transplantation (TGO expressed in per cents).

Tumor's on the 7^{th} day after transplantation.

| Group | Tumor's volume | T% | P |
|---|---|---|---|
| 1 | 85+/-12 | - | - |
| 2 | 45+/-6 | 52% | p<0,001 |
| 3 | 79+/-7 | 7% | p<0,001 |

Results of experiments indicates that single anti-DNA antibodies' administration possess strong antitumor effect. Administration of antibodies fraction that does not contain ant-DNA antibodies do not possess antitumor activity.

### Example 11

### Effect of mice' vaccination by blood plasma DNA fraction received from animals with Erlich carcinoma on transplantation and growth of Erlich carcinoma at immunized animals.

DNA from plasma of mice with Erlich carcinoma was isolated on the 5th day after transplantation according to method mentioned above. Positively charged multilayer liposomes were used as adjuvants for immunization. DNA sample was mixed with liposomes (20 mkg of DNA in 1 mg of lipids).
Group 1 - 6 non immunized mice.
Group 2 - 6 mice immunized for three times with one week interval by intradermal administration of 1 mkg DNA from blood plasma in 50 mkl of liposomal suspension containing 50 mkg of liposomes.
Group 3 - 6 mice immunized for three times with one week interval by intradermal administration of 50 mkl of liposomal suspension containing 50 mkg of liposomes without DNA.
Group 4 - 6 mice immunized for three times with one week interval by intradermal administration of 1 mkg DNA from calf thymus (Sigma) in 50 mkl of liposomal suspension containing 50 mkg of liposomes.

One week after the last immunization Erlih carcinoma was transplanted to all mice including non immunized control mice.

Results of the experiment were estimated according to animal survival on the 30th and 50^{th} day after tumor's transplantation.

| Group | 30 day (number of alive\ number of dead in group) | 50 day (number of alive\ number of dead in group) |
|---|---|---|
| 1 | 0-6 | 0-6 |
| 2 | 5-6 | 3-6 |
| 3 | 0-6 | 0-6 |
| 4 | 2-6 | 0-6 |

So immunization of mice with replanted Erlich carcinoma by blood plasma DNA lead to significant increasing of animals' lifetime.

### Example 12

### DNA isolation from biofilm matrix formed by Gram-positive and Gram-negative bacteria.

Biofilms of Escherichia coli and Staphylococcus aureus were used in experiments. Biofilms were washed off by phosphate buffer from agar. Cells and matrix were separated by centrifuging. Standard phenol-chloroform method was used for DNA extraction from matrix. DNA amount was estimated by spectrophotometric analysis with wave's length 260 nm (SF-46). Received DNA was researched by electrophoresis in 0,8% polyacrylamide gel with ethidium bromide staining.

One part of received DNA was processed for 3 hours by DNAase I at 37°C. After electrophoretic separation of processed and not processed DNA it was found out that:
After electrophoresis non-processed DNA form one compact line. This fact indicates relatively identical size of DNA in biofilm matrix. Said band disappeared completely after DNAse processing. So the biofilm matrix of gram positive and gram negative bacteria contains extracellular DNA, which could be destroyed with DNAse.

### Example 13

### Dynamics of P-glycoprotein expression in Erlich carcinoma at mice that receive Doxorubicin therapy and DNase I effect.

Treatment by Doxorubicin causes expression of P-glycoprotein in tumor tissue that is one of the main MDR (Multidrug Resistance) phenotype mediators. Immunohistochemical staining of mice's tumor histological cuts are listed below.

Mice were subjected to course of 5 day therapy with Doxorubicin (2 mg/kg intravenously daily) or Doxorubicin + DNase I (0,5 mg/kg four times a day during 5 days)

Treatment has begun on the 3^{rd} day after tumor's transplantation. Tissue preparations were executed on the 8th day of tumor's transplantation. Multifocal expression of P-glycoprotein was observed in tumor tissue after 5 days of therapy (Fig.1).

Total level of P-glycoprotein expression and amount of P-glycoprotein positive nodules in tumor tissue was much lower at the case of combined treatment by Doxorubicin + DNase (Fig.1). So treatment by DNase delays development of multidrug resistant phenotype in tumor that is caused by antitumor antibiotic Doxorubicin use.

### Example 14

### Influence of plasma DNA of C57B1 mice with LLC tumor after chemotherapeutical treatment by Doxorubicin, on LLC tumor growth and metastasizes development at C57B1 mice receiving Doxorubicin therapy and effect of DNase I.

LLC tumor was replanted to 30 C57B1 mice. Twenty mice were treated with Doxorubicin at 2 mg/kg dose daily for 5 days, starting day 3 after transplantation. Ten mice were treated with Cyclophosphamide at 15 mg/kg dose intraperitoneally for once on the 3^{rd} day after replantation. Such treatment scheme does not lead to animal's recovery but leads to 50 % tumor inhibition at day 8 in doxorubicin treated animals and 30% tumor inhibition at day 8 in Cyclophosphamide treated animals. On the next day after end of chemotherapy course animals were euthanized and and total blood plasma from both mice groups was taken. After isolation total fraction of blood plasma DNA was stored at -20°C in phosphate buffer.

Five groups of mice that were transplanted with LLC tumor participated in experiment.
Group 1 - 7 mice (control).
Group 2 - 6 mice intravenously treated with Doxorubicin chemotherapy from 3^{rd} up to 8^{th} day at 2 mg/kg dose daily.
Group 3 - 6 mice intravenously treated with Doxorubicin chemotherapy from 3^{rd} up to 8^{th} day at 2 mg/kg dose daily + intravenous administration of DNA fraction from mice previously subjected to Doxorubicin chemotherapy (0,05 mkg of DNA in 200 mkl of phosphate buffer at day 1 and day 3 after initiation of treatment)
Group 4 -6 mice intravenously treated with Doxorubicin chemotherapy from 3^{rd} up to 8^{th} day at 2 mg/kg dose daily + intravenous administration of DNA fraction from mice previously subjected to cyclophosphamide chemotherapy (0,05 mkg of DNA in 200 mkl of phosphate buffer at day 1 and day 3 after initiation of treatment)
Group 5 - 6 mice intravenously treated with Doxorubicin chemotherapy from 3^{rd} up to 8^{th} day at 2 mg/kg dose daily + intravenous administration of DNA fraction from mice previously subjected to Doxorubicin chemotherapy (0,05 mkg of DNA in 200 mkl of phosphate buffer at day 1 and day 3 after initiation of treatment) + intraperitoneal administration of DNase I at 0,5 mg/kg dose for 4 times a day at the first and second days of treatment.

Tumor's size on the 8^{th} day after transplantation.

| Group | Tumor's size |
|---|---|
| 1 | 127+/-13 |
| 2 | 67+/-7 |
| 3 | 115+/-20 |
| 4 | 75+/-11 |
| 5 | 82+/-9 |

So administration of blood plasma DNA from mice subjected to chemotherapy lead to tumor's resistance to chemotherapeutic treatment. DNase's administration prevents appearance of this effect.

### Example 15

### Influence of blood plasma DNA from C57B1 mice with highly metastatic LLC strain on metastasizing of low metastatic LLC tumor strain in C57B1 mice and effect of DNase I.

LLC tumor was transplanted to 30 C57B1 mice. Twenty mice were transplanted with highly metastatic strain and 10 mice were transplanted with low metastatic strain. On the 9th day animals were euthanized and total blood plasma of both mice groups was collected. After isolation the total fraction of blood plasma DNA was stored at -20°C in phosphate buffer.

Five groups of mice with transplanted LLC tumor participated in the experiment.
1 Group - 6 mice transplanted with low metastatic LLC strain.
2 Group - 6 mice transplanted with low metastatic LLC strain + intravenous administration of total DNA fraction from mice with transplanted highly metastatic strain (0,05 mkg of DNA in 200 mkl of phosphate buffer on the 7^{th} and 8^{th} day after transplantation) .
3 Group - 6 mice transplanted with low metastatic LLC strain + intravenous administration of total DNA fraction from mice with transplanted low metastatic strain (0,05 mkg of DNA in 200 mkl of phosphate buffer on the 7^{th} and 8^{th} day after transplantation)
4 Group - 6 mice transplanted with low metastatic LLC strain + intravenous administration of total DNA fraction from mice with transplanted higly metastatic strain (0,05 mkg of DNA in 200 mkl of phosphate buffer on the 7^{th} and 8^{th} day after transplantation) + intraperitoneal administration of DNase I at 1 mg/kg dose two times daily at 7^{th} and 8^{th} day after transplantation.
5 Group - 6 mice transplanted with highly metastatic LLC strain.

Number of metastatic foci in lungs was estimated bon the 15^{th} day after transplantation (N).

Experiments' results are presented in the table.

| Group | N |
|---|---|
| 1 | 12,0 |
| 2 | 24,1 |
| 3 | 14,6 |
| 4 | 11,6 |
| 5 | 33,6 |

Received data indicates that blood plasma DNA from mice with highly metastatic LLC strain intensify metastasizing of low metastatic LLC strain.

DNase administration prevents appearance of this effect.

### Example 16

### DNase I influence on life span of C57B1 mice transplanted with LLC tumor (highly metastatic strain).

Five groups of LLC transplanted mice participated in the experiment.
Group 1 - 7 mice (control).
Group 2- 6 mice were treated with intraperitoneal administration of DNase at 1 mg/kg dose two times a day starting from 3 up to 7 day after tumor transplantation.
Group 3-6 mice were treated with intraperitoneal administration of DNase at 1 mg/kg dose two times a day starting from 3 up to 10 day after tumor transplantation.
Group 4 - 6 mice were treated with intraperitoneal administration of DNase at 1 mg/kg dose two times a day starting from 3 up to 15 day after tumor transplantation.
Group 5 - 6 mice were treated with intraperitoneal administration of DNase at 1 mg/kg dose two times a day starting from 3 up to 18 day after tumor transplantation.

Results of experiment were estimated according to animals' survival on the 30 and 50 day after tumor transplantation.

| Group | 30 day (number of alive\ number of dead in group) | 50 day (number of alive\ number of dead in group) |
|---|---|---|
| 1 | 0-7 | 0-7 |
| 2 | 0-6 | 0-6 |
| 3 | 3-6 | 0-6 |
| 4 | 5-1 | 3-3 |
| 5 | 6-0 | 6-0 |

The significant inhibition of tumor's growth was observed at the last day of DNase treatment in the 2nd and 3rd groups, but tumor's growth renewed after DNase withdrawal and to the 25^{th} day size of tumor in this groups and in control has equalized.

The most longitudinal course o DNase treatment (from 3^{rd} up to 18^{th} day - group number 6) has lead to maximal survival. Inhibition of tumor growth was more than 95% at day 18.

In all experiments single and multiple injection of up to 2,5 mg/kg of human DNase I (maximal dose that was used in experiments) had no toxic effect on animals.

So, DNase I does not cause direct cytotoxic effect on tumor cells (in our in vitro experiments at concentration of 100 mkg/ml) and experimental data confirm that antitumor effect is connected with destruction of DNA in blood plasma and DNase's therapeutic effect increases with increasing of its treatment course duration.

### Example 17

### Influence of different methods of blood plasma DNA's destruction, inactivation, and binding on ability of blood plasma DNA from C57B1 mice with transplanted highly metastatic LLC strain to intensify metastasizing of low metastatic LLC tumor strain in C57BI mice.

100 mice were transplanted with highly metastatic LLC strain. On the 9th day after transplantation, animals were euthanized and total blood plasma was taken. After isolation total fraction of blood plasma DNA was stored at -20°C in phosphate buffer.

Six groups of mice with transplanted low metastatic LLC strain participated in the experiment.
Group 1 - 6 mice transplanted with low metastatic LLC strain.
Group 2 - 6 mice transplanted with low metastatic LLC strain + two intravenous injections of total DNA fraction from mice transplanted with highly metastatic strain on the 7^{th} and 8^{th} day after transplantation(0,05 mkg of DNA was dissolved in 500 mkl of fresh heparinized blood before injection).
Group 3 - 6 mice transplanted with low metastatic LLC strain + two intravenous injections of total DNA fraction from mice transplanted with highly metastatic strain on the 7^{th} and 8^{th} day after transplantation(0,05 mkg of DNA was dissolved in 500 mkl of fresh blood plasma before injection). Before administration the sample was photochemically disinfected (1 mkM of methylene blue was added with following irradiation by red light during 10 minutes (60 000 Lux).
Group 4 - 6 mice transplanted with low metastatic LLC strain + two intravenous injections of total DNA fraction from mice transplanted with highly metastatic strain on the 7^{th} and 8^{th} day after transplantation(0,05 mkg of DNA was dissolved in 500 mkl of fresh blood plasma before injection). The sample was passed through the column containing DEAE-cellulose for two times before administration.
Group 5 - 6 mice transplanted with low metastatic LLC strain + two intravenous injections of total DNA fraction from mice transplanted with highly metastatic strain on the 7^{th} and 8^{th} day after transplantation(0,05 mkg of DNA was dissolved in 500 mkl of fresh heparinized blood before injection. 1 mkg of fragment A of Ricin toxin was added to the sample before administration and sample was incubated at 370C for 1 hour. Ricin toxin is representative of RIP toxins family (proteins that inactivate ribosomes) which are used for immunotoxin's creation. Besides their ability to inactivate ribosomes these proteins can **deadenylate** DNA. For realization of toxic effect catalytic subunit A of RIP II type should by delivered to cell by B subunit. Without B subunit A chain is not toxic but can be used for blood plasma DNA's inactivation due to its polynucleotide-adenylglicozidase activity.
Group 6-6 mice transplanted with low metastatic LLC strain + two intravenous injections of total DNA fraction from mice transplanted with highly metastatic strain on the 7^{th} and 8^{th} day after transplantation(0,05 mkg of DNA was dissolved in 500 mkl of fresh heparinized blood before injection. Total DNA fraction was enzymatically methylated before administration (I.Muiznieks et.al.,FEBS Letters,1994,v.344,pp.251-254).

Number of metastaic nodules in lungs was estimated on the 15th day after transplantation.

Results of the experiments are presented in the table.

| Group | Ncp. |
|---|---|
| 1 | 12,0 |
| 2 | 22,5 |
| 3 | 14,1 |
| 4 | 15,5 |
| 5 | 15,1 |
| 6 | 12,3 |

Received data indicates that all used methods inhibited ability of blood plasma DNA of mice with highly metastatic LLC tumor strain to increase metastasizing process of low metastatic LLC tumor strain.

### Example 18

### Influence of DNase I therapy on the development of somatic mosaicism.

Frequency of HPRT gene's mutations in blood T-lymphocytes was studied as a model of somatic mosaicism. Human HPRT gene (chromosome Xq26) encodes enzyme that is constitutionally expressing but not essential and is involved in metabolism of purine nucleotides. Cloning was done according to Bigbee W. method (Bigbee W. Et al., Mutation Res.,1998,v.397,pp.119-136).

Lymphocytes from peripheral blood of 8 patients subjected to 3 week course of immunostimulating therapy by Neovir after surgical elimination of tumor were used for cloning. 4 patients out from 8 were additionally subjected to intravenous administration of human recombinant DNase I (200 mkg/kg dose 4 times a day during three weeks).

Incidence of HPRT - deficient clones in the blood of patients that received DNase I therapy was 3 times less than in blood of patients that received only immunostimulating therapy.

### Example 19

### Influence of DNase I therapy on old rats' life time.

White not thoroughbred 24 month old rats were used in experiment. In the study group (10 animals) rats starting from the age of 24 months were subjected to intravenous administration of polysialated human DNase I at 500 mg/kg (equivalent dose of non-modified enzyme) 2 times a week during 2 months. Control group of animals got the administration of placebo. Average life time of rats from control group was 27,8 months. Average life time of rats from experimental group was 30,1 months.

Example 20

Influence of DNase I therapy on viability of pancreatic 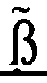 cells and endothelium of aorta.

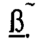 cells of human embryonic pancreas and endothelial cells of human aorta were used for primary cell culture formation. DNA isolated from plasma of patient with severe 11-type diabetes complicated by systemic atherosclerosis (0,0025 mkg per 1 ml of culture media) was added to cell culture 24 hours after passage.

In other experimental series DNA of the patient before introduction to cell culture was processed with enzymatic methylation. Amount of viable cells was estimated 24 hours later with trypan blue staining.

Results of experiment are presented in the table.

Percentage of viable cells 48 hours after subculturing.

| Cells | Control | Patient's DNA | Methylated DNA |
|---|---|---|---|
| B-cells | 73% | 43% | 61% |
| Endothelium | 62% | 35% | 55% |

### Industrial applicability

Current invention justify that destruction (binding, inactivation) of circulating blood plasma DNA in course of oncological and bacterial diseases possess significant therapeutic effect.

Invention is confirmed by the fact that blood plasma DNA of patients contains unique genes that takes part in formation and maintenance of tumor's "malignant phenotype".

Binding, destruction or inactivation of DNA circulating in blood plasma possess therapeutic effect at diseases which development is connected with accumulation and spreading of somatic mutations in host's cells.

Blood plasma circulating DNA can be destructed, inactivated or eliminated from blood plasma by DNases, sorbents, antibodies and other methods which provide inactivation by destroying, binding or modification of circulating DNA.

Treatment directed to the destruction of blood plasma DNA lead to significant atitumor effect while its own toxicity is immaterial.

Treatment directed to the destruction of blood plasma DNA if combined with traditional antitrumor therapy lead to significant antitumor effect.

Efficiency of the treatment directed to blood plasma DNA's destruction can be estimated by monitoring of DNA amount and amount of tumor's genetic markers in blood plasma of patient who receive such therapy.

Cloning and sequencing of blood plasma DNA from oncological patient's can be used for studying of genetic processes that take part in oncogenesis and identification of new genes connected with oncogenesis processes.

Cloning and sequencing of healthy people's blood plasma DNA can be used for studying of genome's operating in healthy individual and in the development of somatic diseases and in identification of new genes that are involved in these processes.

Pharmaceutical compositions containing components that inactivate by destroying, binding and modification the blood plasma DNA could be used for treatment of patients with oncological diseases, infections, somatic diseases and for prolongation of life time.

For providing effective therapeutic exposition of active component that is necessary for blood plasma DNA' destruction, and for therapeutic effect achievement pharmaceutically acceptable compositions and modifications, drug delivery systems are used providing maximal systemic circulations of the active component in blood plasma and its contact with DNA circulating in plasma. The main way of administration is intravenous. But other ways of administration that provide coming of active component in to systemic circulation, such as subcutaneous, intramuscular, inhalation, intraperitoneal and others can also be used. Doses and regimes of administration are determined by nature of used active ingredient and way of administration. The effect is controlled by the level of DNA's contents and its dynamics in blood plasma, presence of tumor, infectious and other genetic markers, and appearing of positive clinical dynamics of disease.

## Claims

1. Treatment method of oncological and/or infectious and/or somatic diseases by acting on biological targets inside organisms, differs in that the biological target is extratcellular DNA including extracellular DNA circulating in blood plasma.

2. Treatment method of oncological and/or infectious and/or somatic diseases according claim 1 differs in that the extratcellular DNA is inactivated by destruction, binding or modification.

3. Treatment method of oncological and/or infectious and/or somatic diseases according claim land 2 , differs in that the extratcellular DNA is inactivated by destruction, binding or modification by injecting to patient of pharmaceutical agent which is capable to destroy, bind or modify free circulating DNA.

4. Treatment method of oncological and/or infectious and/or somatic diseases according claim 1-3 , differs in that the extratcellular DNA is inactivated by destruction, binding or modification by pharmaceutical agent's injection in amount sufficient for destruction and in therapeutic regime providing destruction, binding or modification in sufficient for therapeutic effect achievement period of time.

5. Treatment method of oncological and/or infectious and/or somatic diseases according claim 1-4 differs in that the genetically modified cells or genotherapeutic constructions are injected to patient when said remedies induce synthesis in host's organism of biopolymers, capable to inactivate free circulating blood plasma DNA by its binding, destruction or modification.

6. Treatment method of oncological and/or infectious and/or somatic diseases according claim 1 or 2, differs in that the circulating extracellular DNA is inactivated by destruction, binding or modification using extracorporeal blood processing.

7. Treatment method of oncological and/or infectious and/or somatic diseases according claim 1,2 or 6, differs in that the extracorporal purification of patient's blood from free circulating DNA is achieved by immune or affine absorption.

8. Treatment method of oncological and/or infectious and/or somatic diseases according claim 1,2 or 6, differs in that the extracorporal purification of patient's blood from free circulating DNA is achieved by methods of gravitational blood surgery.

9. Treatment method of oncological and/or infectious and/or somatic diseases according claim 1,2 or 6, differs in that the extracorporal purification of patient's blood from free circulating DNA is achieved by biological, chemical or photochemical inactivation.

10. Treatment method of oncological and/or infectious and/or somatic diseases according claim 1 or 2, differs in that the patient is immunized by vaccine , which vaccine contain blood plasma circulating DNA (including said DNA with naturally complexed proteins) as the antigen..

11. Treatment method of oncological and/or infectious and/or somatic diseases according claims 1-10 , differs in that the treatment is combined with surgical, chemiotherapeutic, hormonal, radiation and immunotherapeutic methods.

12. Pharmaceutical agent for oncological and/or infectious and/or somatic disease treatment, representing compound possessing desoxyribonuclease activity and/or being able to inactivate extracellular DNA including DNA circulating in patients blood plasma.

13. Pharmaceutical agent according claim 12 differs in that compound possessing desoxyribonuclease activity is desoxyribonuclease enzyme.

14. Pharmaceutical agent according claim 13 differs in that desoxyribonuclease is modified for better pharmacodynamic and pharmacokinetic performance and comprises desoxyribonuclease analogue with increased activity, desoxyribonuclease analogue not sensitive to endogenous inhibitors of desoxyribonuclease, polysialated desoxyribonuclease, pegylated desoxyribonuclease, desoxyribonuclease that is bound or mixed with synthetic and natural microspheres, liposomes, dextran, and other corpuscular natural and synthetic polymer carriers.

15. Pharmaceutical agent according claims 12-14 which additionally contains ribonuclease and/or lipase and/or proteinase.

16. Pharmaceutical agent according claim 12, differs in that the compound possessing desoxyribonuclease activity is antibody possessing nuclease activity, in particular polyclonal DNA- abzymes, monoclonal DNA-abzymes or their derivatives.

17. Pharmaceutical agent according claim 12, differs in that the compound able to bind DNA is antibody able to bind DNA and its complexes and derivatives of said antibody.

18. Pharmaceutical composition for oncological and infectious diseases treatment, containing pharmaceutical agent according claims . 12-16 in therapeuticcaly effective amount and pharmaceutically acceptable carrier or excipient.

19. Method to increase the life time which is achieved by inactivation of extracellular DNA circulating in blood plasma by said DNA destruction, binding or modification according claims 2-17.

20. Method of prophylaxis of pathologies connected with appearance and development of somatic mosaicismby the way of destruction, binding or modification of DNA according to claims 2-17.

21. Method to control the treatment efficacy of oncological and/or infectious and somatic diseases , to estimate the infection development , to control the efficacy of treatment directed to prolongation of life time , by the way of measurement of patient biochemical factors, differs in that monitoring for control of such treatment sizes of molecules, fractions' correlation, bindings with proteins, lipids and sugars, nucleotide consequences of free circulating blood plasma DNA are used.

22. Usage of blood plasma DNA and extracellular microbial DNA for evaluation of DNA involved in process of diseases' appearance and development, which usage includes its cloning, sequencing, identification of genes, unique and repeated sequences with their future studying.
